# EUROPÄISCHE PATENTSCHRIFT

(11) **EP 3 679 131 B1**
(45) Veröffentlichungstag und Bekanntmachung des Hinweises auf die Patenterteilung: **04.09.2024**
(21) Anmeldenummer: 18765856.2
(22) Anmeldetag: 05.09.2018
(51) Int. Cl.: C12N 9/54, C11D 3/386

(54) **LEISTUNGSVERBESSERTE PROTEASEVARIANTEN II**
PERFORMANCE-ENHANCED PROTEASE VARIANTS II
VARIANTES DE PROTÉASES À PERFORMANCES AMÉLIORÉES

(30) Priorität: 05.09.2017 DE 102017215631; 05.06.2018 DE 102018208777
(43) Veröffentlichungstag der Anmeldung: 15.07.2020
(73) Patentinhaber: Henkel AG & Co. KGaA, 40589 Düsseldorf (DE)
(72) Erfinder: FERNANDEZ, Layla, 50858 Köln (DE); GRIEMERT, Sabine, 40789 Monheim am Rhein (DE); MUSSMANN, Nina, 47877 Willich (DE); WIELAND, Susanne, 41541 Zons/Dormagen (DE); HERBST, Daniela, 40211 Düsseldorf (DE); PRUESER, Inken, 40215 Düsseldorf (DE); DEGERING, Christian, 40699 Erkrath (DE); STRAUSS, Daria, 40627 Düsseldorf (DE); EGGERT, Thorsten, 45529 Hattingen (DE); LEGGEWIE, Christian, 45468 Mülheim an der Ruhr (DE)
(86) Internationale Anmeldenummer: PCT/EP2018/073880
(87) Internationale Veröffentlichungsnummer: WO 2019/048486

(56) Entgegenhaltungen:
- WO-A1-2011/036263
- WO-A1-2017/162429
- WO-A1-2017/198488
- DE-A1- 102006 022 224
- "RecName: Full=Subtilisin Carlsberg; EC=3.4.21.62; Flags: Precursor", UNIPROT,, 21 July 1986 (1986-07-21), XP002769870
- PHILIP N. BRYAN: "Protein engineering of subtilisin", BIOCHIMICA ET BIOPHYSICA ACTA. PROTEIN STRUCTURE AND MOLECULAR ENZYMOLOGY, vol. 1543, no. 2, 1 December 2000 (2000-12-01), AMSTERDAM; NL, pages 203 - 222, XP055251064, ISSN: 0167-4838, DOI: 10.1016/S0167-4838(00)00235-1
- VON DER OSTEN C ET AL: "Protein engineering of subtilisins to improve stability in detergent formulations", JOURNAL OF BIOTECHNOLOGY, ELSEVIER, AMSTERDAM, NL, vol. 28, no. 1, 1 March 1993 (1993-03-01), pages 55 - 68, XP023944106, ISSN: 0168-1656, [retrieved on 19930301], DOI: 10.1016/0168-1656(93)90125-7
- LJUBICA VOJCIC ET AL: "Advances in protease engineering for laundry detergents", NEW BIOTECHNOLOGY, vol. 32, no. 6, 1 December 2015 (2015-12-01), NL, pages 629 - 634, XP055327595, ISSN: 1871-6784, DOI: 10.1016/j.nbt.2014.12.010

## Beschreibung

Die Erfindung liegt auf dem Gebiet der Enzymtechnologie. Die Erfindung betrifft Proteasen aus *Bacillus pumilus,* deren Aminosäuresequenz, insbesondere im Hinblick auf den Einsatz in Wasch- und Reinigungsmitteln verändert wurden, um ihnen eine bessere Lagerstabilität zu verleihen, und die für sie codierende Nukleinsäuren sowie deren Herstellung. Die Erfindung betrifft ferner die Verwendungen dieser Proteasen und Verfahren, in denen sie eingesetzt werden, sowie diese enthaltende Mittel, insbesondere Wasch- und Reinigungsmittel.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R. Siezen, Seite 75-95 in "Subtilisin enzymes", herausgegeben von R. Bott und C. Betzel, New York, 1996. Subtilasen werden natürlicherweise von Mikroorganismen gebildet. Hierunter sind insbesondere die von Bacillus-Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die Protease aus *Bacillus lentus,* insbesondere aus *Bacillus lentus* DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so beispielsweise für den Einsatz in Wasch- und Reinigungsmitteln optimiert. Dazu gehören Punkt-, Deletions- oder Insertionsmutagenese oder Fusion mit anderen Proteinen oder Proteinteilen. So sind für die meisten aus dem Stand der Technik bekannten Proteasen entsprechend optimierte Varianten bekannt. Aus WO 2011/036263 sind zum Beispiel Savinase und/oder BPN' Varianten bekannt.

In EP 2016175 A1, WO 2017/198488 und WO 2017/162429 sind eine für Wasch- und Reinigungsmittel vorgesehene Protease aus *Bacillus pumilus* offenbart. Generell sind nur ausgewählte Proteasen für den Einsatz in flüssigen Tensid-haltigen Zubereitungen überhaupt geeignet. Viele Proteasen zeigen in derartigen Zubereitungen keine ausreichende katalytische Leistung. Für die Anwendung von Proteasen in Reinigungsmitteln ist daher eine hohe katalytische Aktivität unter Bedingungen wie sie sich während eines Waschgangs darstellen und eine hohe Lagerstabilität besonders wünschenswert.

Folglich haben Protease- und Tensid-haltige flüssige Formulierungen aus dem Stand der Technik den Nachteil, dass die enthaltenen Proteasen unter Standard-Waschbedingungen (z.B. in einem Temperaturbereich von 20°C bis 40°C) keine zufriedenstellende proteolytische Aktivität aufweisen oder nicht lagerstabil sind und die Formulierungen daher keine optimale Reinigungsleistung an Protease-sensitiven Anschmutzungen zeigen.

Überraschenderweise wurde jetzt festgestellt, dass eine Protease aus *Bacillus pumilus* oder eine hierzu hinreichend ähnliche Protease (bezogen auf die Sequenzidentität), die Aminosäuresubstitutionen (a) an den Positionen, die den Positionen 9 und 271, jeweils bezogen auf die Nummerierung gemäß SEQ ID NO:1, entsprechen, und (b) an mindestens drei der Positionen, die den Positionen 29, 48, 101, 130, 131, 133, 144, 224 oder 252, jeweils bezogen auf die Nummerierung gemäß SEQ ID NO:1, entsprechen, aufweist, hinsichtlich der Lagerstabilität gegenüber der Wildtypform und/oder Referenzmutanten verbessert ist und daher besonders für den Einsatz in Wasch- oder Reinigungsmitteln geeignet ist.

Gegenstand der Erfindung ist daher in einem ersten Aspekt eine Protease gemäß Anspruch 1, umfassend eine Aminosäuresequenz, die mindestens 80% Sequenzidentität mit der in SEQ ID NO:1 angegebenen Aminosäuresequenz über deren Gesamtlänge aufweist und (i) an den Positionen, die den Positionen 9 und 271 gemäß SEQ ID NO:1 entsprechen Aminosäuresubstitutionen aufweist, vorzugsweise die Aminosäuresubstitutionen 9T, 9H, 9S oder 9A und 271E, und (ii) eine Aminosäuresubstitution an mindestens drei der Positionen, die den Positionen 29, 48, 101, 130, 131, 133, 144, 224 oder 252, jeweils bezogen auf die Nummerierung gemäß SEQ ID NO:1, entsprechen, aufweist,
wobei
(1) die Aminosäuresubstitutionen an den Positionen, die den Positionen 9 und 271 entsprechen, die Aminosäuresubstitutionen P9T, P9H, P9S oder P9A und Q271E sind; und/oder,
(2) die mindestens eine Aminosäuresubstitution gemäß (b) aus der Gruppe bestehend aus A29G, A48V, D101E, N130D, N130S, N130H, G131S, G131N, G131D, G131K, T133K, T133R, T133Y, N144K, N144L, N144A, S224A, S224T oder N252S, jeweils bezogen auf die Nummerierung gemäß SEQ ID NO:1, ausgewählt ist.

In einer Ausführungsform dieses ersten Aspekts betrifft die Erfindung eine Protease umfassend eine Aminosäuresequenz, die mindestens 80% Sequenzidentität mit der in SEQ ID NO:1 angegebenen Aminosäuresequenz über deren Gesamtlänge aufweist und jeweils bezogen auf die Nummerierung gemäß SEQ ID NO:1 aufweist:
(a) an den Positionen, die den Positionen 9, 130, 144, 252 und 271 entsprechen, Aminosäuresubstitutionen, insbesondere die Aminosäuresubstitutionen 9T, 130D, 144K, 252T und 271E; sowie optional
(b1) an der Position, die der Position 166 entspricht, die Aminosäuresubstitution 166M, 166Q oder 166A; und/oder
(b2) an mindestens einer der Positionen, die den Positionen 62, 133, 149, 156, 172 oder 217 entsprechen, mindestens eine weitere Aminosäuresubstitution.

In einem zweiten Aspekt betrifft die Erfindung auch eine Protease umfassend eine Aminosäuresequenz, die mindestens 80% Sequenzidentität mit der in SEQ ID NO:1 angegebenen Aminosäuresequenz über deren Gesamtlänge aufweist und bezogen auf die Nummerierung gemäß SEQ ID NO:1 an mindestens einer der Positionen, die den Positionen 62, 149, 156, 166, 172 oder 217 entsprechen, mindestens eine Aminosäuresubstitution aufweist, wobei die Aminosäuresubstitution an der Position, die Position 166 entspricht, ausgewählt wird aus 166M, 166Q und 166A, und die Aminosäuresubstitution an der Position, die Position 62 entspricht, ausgewählt wird aus 62S.

In einem weiteren Aspekt bezieht sich die vorliegende Erfindung auf eine Protease, die eine Aminosäuresequenz, die mindestens 80% Sequenzidentität mit der in SEQ ID NO:1 angegebenen Aminosäuresequenz über deren Gesamtlänge aufweist und die mindestens drei Aminosäuresubstitutionen umfasst, die aus der Gruppe bestehend aus N130D, N130S, N130H, G131N, G131D, G131K, T133K, T133Y, N144L, N144A, S224A, oder N252S, jeweils bezogen auf die Nummerierung gemäß SEQ ID NO:1, ausgewählt sind. In einer solchen Ausführungsform weist die Protease eine Substitution an einer der Positionen 9 und 271 sowie optional zusätzlich eine Substitution an der Position 216.

In verschiedenen Ausführungsformen der oben beschriebenen Aspekte kann die Sequenzidentität mit SEQ ID NO:1 vorzugsweise 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, oder 98,8% betragen.

Ein weiterer Gegenstand der Erfindung ist ein Verfahren zur Herstellung einer Protease umfassend das Substituieren von Aminosäuren in einer Ausgangsprotease, die mindestens 80% Sequenzidentität zu der in SEQ ID NO:1 angegebenen Aminosäuresequenz über deren Gesamtlänge aufweist (i) an den Positionen, die Positionen 9 und 271 in SEQ ID NO:1 entsprechen, vorzugsweise derart, dass die Protease an den Positionen die Aminosäuresubstitutionen 9T, 9H, 9S oder 9A, insbesondere 9T, und 271E umfasst, und (ii) an mindestens drei der Positionen, die den Positionen 29, 48, 101, 130, 131, 133, 144, 224 oder 252 in SEQ ID NO:1 entsprechen, vorzugsweise derart, dass die Protease mindestens drei der Aminosäuresubstitutionen 29G, 48V, 101E, 130D, 130S, 130H, 131S, 131N, 131D, 131K, 133K, 133R, 133Y, 144K, 144L, 144A, 224A, 224T, 252T oder 252S aufweist. Die mittels diesem Verfahren erhältliche Protease weist vorzugsweise mindestens 80% Sequenzidentität zu der in SEQ ID NO:1 angegebenen Aminosäuresequenz über deren Gesamtlänge auf.

In verschiedenen Ausführungsformen dieses Aspekts betrifft die Erfindung ein Verfahren zur Herstellung einer wie oben definierten Protease umfassend das Substituieren von Aminosäuren in einer Ausgangsprotease, die mindestens 80% Sequenzidentität zu der in SEQ ID NO:1 angegebenen Aminosäuresequenz über deren Gesamtlänge aufweist (i) an den Positionen, die den Positionen 9, 130, 144, 252 und 271 in SEQ ID NO:1 entsprechen, derart, dass die Protease an den Positionen Aminosäuresubstitutionen, insbesondere die Aminosäuresubstitutionen 9T, 130D, 144K, 252T und 271E umfasst, sowie optional (ii) an der Position, die der Position 166 in SEQ ID NO:1 entspricht, derart, dass die Protease mindestens eine der Aminosäuresubstitutionen 166M, 166Q oder 166A und/oder (iii) an mindestens einer der Positionen, die den Positionen 62, 133, 149, 156, 172 oder 217 in SEQ ID NO:1 entsprechen, mindestens eine weitere Aminosäuresubstitution aufweist. Die mittels diesem Verfahren erhältliche Protease weist mindestens 80% Sequenzidentität zu der in SEQ ID NO:1 angegebenen Aminosäuresequenz über deren Gesamtlänge auf.

Eine Protease im Sinne der vorliegenden Patentanmeldung umfasst daher sowohl die Protease als solche als auch eine mit einem erfindungsgemäßen Verfahren hergestellte Protease. Alle Ausführungen zur Protease beziehen sich daher sowohl auf die Protease als solche wie auch auf die mittels entsprechender Verfahren hergestellten Proteasen.

Weitere Aspekte der Erfindung betreffen die für diese Proteasen codierenden Nukleinsäuren, erfindungsgemäße Proteasen oder Nukleinsäuren enthaltende nicht menschliche Wirtszellen sowie erfindungsgemäße Proteasen umfassende Mittel, insbesondere Wasch- und Reinigungsmittel, Wasch- und Reinigungsverfahren, und Verwendungen der erfindungsgemäßen Proteasen in Wasch- oder Reinigungsmitteln zur Entfernung von proteinhaltigen Anschmutzungen.

"Mindestens eine", wie hierin verwendet, bedeutet eine oder mehrere, d.h. 1, 2, 3, 4, 5, 6, 7, 8, 9, 10, 11, 12, 13, 14 oder mehr.

Wenn hierin die Protease derart definiert wird, dass sie "die Aminosäuresubstitutionen 9T, 9H, 9S oder 9A" umfasst, bedeutet dies, dass die Position 9 entweder zu T, H, S oder A mutiert ist. Der Ausdruck, dass die Protease "die Aminosäuresubstitutionen 9T, 9H, 9S oder 9A und 271E sowie optional 216C" umfasst, bedeutet somit, dass die Position 9 entweder zu T, H, S oder A mutiert, die Position 271 zu E und die Position 216 optional zu C mutiert ist.

Die vorliegende Erfindung basiert auf der überraschenden Erkenntnis der Erfinder, dass Aminosäuresubstitutionen an den Positionen, die den Positionen 9 und 271, jeweils bezogen auf die Nummerierung gemäß SEQ ID NO:1, entsprechen, derart, dass die Protease an den Positionen die Aminosäuresubstitutionen 9T, 9H, 9S oder 9A und 271E umfasst, und eine Aminosäuresubstitution an mindestens drei der Positionen, die den Positionen 29, 48, 101, 130, 131, 133, 144, 224 oder 252 der Protease aus *Bacillus pumilus* gemäß SEQ ID NO:1 entsprechen, in einer Protease, die eine zu der in SEQ ID NO:1 angegebenen Aminosäuresequenz zu mindestens 80% identische Aminosäuresequenz umfasst, derart, dass an mindestens drei der entsprechenden Positionen die Aminosäuren 29G, 48V, 101E, 130D, 130S, 130H, 131S, 131N, 131D, 131K, 133K, 133R, 133Y, 144K, 144L, 144A, 224A, 224T, 252T oder 252S vorhanden sind, eine verbesserte Lagerstabilität dieser veränderten Protease in Wasch- und Reinigungsmitteln bewirkt. Es wurde weiterhin gefunden, dass diese Lagerstabilität in Proteasen auf Basis der Sequenz gemäß SEQ ID NO:1, die an den Positionen, die den Positionen 9, 130, 144, 252 und 271 entsprechen, Aminosäuresubstitutionen, insbesondere die Aminosäuresubstitutionen 9T, 130D, 144K, 252T und 271E; sowie optional (b1) an der Position, die der Position 166 entspricht, die Aminosäuresubstitution 166M, 166Q oder 166A; und/oder (b2) an mindestens einer der Positionen, die den Positionen 62, 133, 149, 156, 172 oder 217 entsprechen, mindestens eine weitere Aminosäuresubstitution aufweisen, in besonders vorteilhafter Weise verbessert ist. Das ist insbesondere insoweit überraschend, als dass keine der oben genannten Aminosäuresubstitutionen zuvor mit einer erhöhten Lagerstabilität der Protease in Verbindung gebracht wurde. Optional kann auch zusätzlich eine verbesserte Leistung dieser veränderten Protease (proteolytische Aktivität unter Standard-Waschbedingungen) in Wasch- und Reinigungsmitteln bewirkt werden.

In verschiedenen Ausführungsformen der erfindungsgemäßen Protease weist die Protease Aminosäuresubstitutionen
(A) (i) an den Positionen, die den Positionen 9, 130, 133 und 271, jeweils bezogen auf die Nummerierung gemäß SEQ ID NO:1, entsprechen, und optional (ii) an mindestens einer der Positionen, die den Positionen 29, 48, 101, 131, 144, 224 oder 252, jeweils bezogen auf die Nummerierung gemäß SEQ ID NO:1, entsprechen, auf;
(B) (i) an den Positionen, die den Positionen 9, 130, 144 und 271, jeweils bezogen auf die Nummerierung gemäß SEQ ID NO:1, entsprechen, und optional (ii) an mindestens einer der Positionen, die den Positionen 29, 48, 101, 131, 133, 224 oder 252, jeweils bezogen auf die Nummerierung gemäß SEQ ID NO:1, entsprechen, auf;
(C) (i) an den Positionen, die den Positionen 9, 130, 252 und 271, jeweils bezogen auf die Nummerierung gemäß SEQ ID NO:1, entsprechen, und optional (ii) an mindestens einer der Positionen, die den Positionen 29, 48, 101, 131, 133, 144 oder 224, jeweils bezogen auf die Nummerierung gemäß SEQ ID NO:1, entsprechen, auf;
(D) (i) an den Positionen, die den Positionen 9, 133, 144 und 271, jeweils bezogen auf die Nummerierung gemäß SEQ ID NO:1, entsprechen, und optional (ii) an mindestens einer der Positionen, die den Positionen 29, 48, 101, 130, 131, 224 oder 252, jeweils bezogen auf die Nummerierung gemäß SEQ ID NO:1, entsprechen, auf;
(E) (i) an den Positionen, die den Positionen 9, 133, 252 und 271, jeweils bezogen auf die Nummerierung gemäß SEQ ID NO:1, entsprechen, und optional (ii) an mindestens einer der Positionen, die den Positionen 29, 48, 101, 130, 131, 144 oder 224, jeweils bezogen auf die Nummerierung gemäß SEQ ID NO:1, entsprechen, auf;
(F) (i) an den Positionen, die den Positionen 9, 144, 252 und 271, jeweils bezogen auf die Nummerierung gemäß SEQ ID NO:1, entsprechen, und optional (ii) an mindestens einer der Positionen, die den Positionen 29, 48, 101, 130, 131, 133, oder 224, jeweils bezogen auf die Nummerierung gemäß SEQ ID NO:1, entsprechen, auf;
(G) (i) an den Positionen, die den Positionen 9, 130, 144, 252 und 271, jeweils bezogen auf die Nummerierung gemäß SEQ ID NO:1, entsprechen, und optional (ii) an mindestens einer der Positionen, die den Positionen 29, 48, 101, 131, 133, oder 224, jeweils bezogen auf die Nummerierung gemäß SEQ ID NO:1, entsprechen, auf;
(H) (i) an den Positionen, die den Positionen 9, 133, 144, 252 und 271, jeweils bezogen auf die Nummerierung gemäß SEQ ID NO:1, entsprechen, und optional (ii) an mindestens einer der Positionen, die den Positionen 29, 48, 101, 130, 131, oder 224, jeweils bezogen auf die Nummerierung gemäß SEQ ID NO:1, entsprechen, auf;
(I) (i) an den Positionen, die den Positionen 9, 130, 133, 252 und 271, jeweils bezogen auf die Nummerierung gemäß SEQ ID NO:1, entsprechen, und optional (ii) an mindestens einer der Positionen, die den Positionen 29, 48, 101, 131, 144, oder 224, jeweils bezogen auf die Nummerierung gemäß SEQ ID NO:1, entsprechen, auf;
(J) (i) an den Positionen, die den Positionen 9, 130, 133, 144, und 271, jeweils bezogen auf die Nummerierung gemäß SEQ ID NO:1, entsprechen, und optional (ii) an mindestens einer der Positionen, die den Positionen 29, 48, 101, 131, 224, oder 252, jeweils bezogen auf die Nummerierung gemäß SEQ ID NO:1, entsprechen, auf;
(K) (i) an den Positionen, die den Positionen 9, 130, 133, 144, 252 und 271, jeweils bezogen auf die Nummerierung gemäß SEQ ID NO:1, entsprechen, und optional (ii) an mindestens einer der Positionen, die den Positionen 29, 48, 101, 131, oder 224, jeweils bezogen auf die Nummerierung gemäß SEQ ID NO:1, entsprechen, auf; oder
(L) (i) an den Positionen, die den Positionen 9, 130, 144, 252 und 271 entsprechen, Aminosäuresubstitutionen, insbesondere die Aminosäuresubstitutionen 9T, 130D, 144K, 252T und 271E; und (ii) an einer der Positionen, die den Positionen 62, 149, 156, 166, 172 oder 217, entsprechen, eine der Aminosäuresubstitutionen 62S, 149I, 156G, 156Q, 166M, 166Q, 166A, 172P, 172G oder 217M auf.

In weiteren Ausführungsformen sind bevorzugte Proteasen solche mit Aminosäuresubstitutionen an den Positionen:
9 und 271 sowie mindestens einer von 131, 133, 224, 130 und 144 sowie optional mindestens einer von 29, 48, 101 und 252;
9 + 271 +131 + 224 und optional mindestens eine von 29, 48, 101, 130, 133, 144 oder 252;
9 + 271 +131 + 130 und optional mindestens eine von 29, 48, 101, 130, 144, 224 oder 252;
9 + 271 +131 + 130 + 224 und optional mindestens eine von 29, 48, 101, 130, 144 oder 252; oder
9 + 271 +131 + 133 + 224 +130 und optional mindestens eine von 29, 48, 101, 144 oder 252;

Hierbei sind die entsprechenden Austausche insbesondere die oben erwähnten, d.h. 9T, 9H, 9S, 9A, 29G, 48V, 101E, 130D, 130S, 130H, 131D, 131N, 131S, 131K, 133K, 133R, 133Y, 144K, 144L, 144A, 224A, 224T, 252S, 252T und/oder 271E.

In verschiedenen Ausführungsformen weist die Protease
(1) an den Positionen, die den Positionen 9, 130, 144, 252 und 271 entsprechen, Aminosäuresubstitutionen, insbesondere die Aminosäuresubstitutionen 9T, 130D, 144K, 252T und 271E; und
(2) an einer der Positionen, die den Positionen 62, 149, 156, 166, 172 oder 217, entsprechen, eine der Aminosäuresubstitutionen 62S, 149I, 156G, 156Q, 166M, 166Q, 166A, 172P, 172G oder 217M;

In bevorzugten Ausführungsformen der erfindungsgemäßen Protease weist die Protease an den Positionen, die den Positionen 149, 156, 172 oder 217 entsprechen, Substitutionen auf, die ausgewählt sind aus den Aminosäuresubstitutionen 149I, 156G, 156Q, 172P, 172G und 217M.

In verschiedenen Ausführungsformen weist die Protease an den Positionen, die den Positionen 9, 130, 144, 252 und 271 entsprechen, Aminosäuresubstitutionen, insbesondere die Aminosäuresubstitutionen 9T, 130D, 144K, 252T und 271E; und an einer oder mehreren der Positionen, die den Positionen 62, 149, 156, 166, 172 oder 217, entsprechen, mindestens eine, beispielsweise 1, 2, 3, 4, 5 oder 6, beispielsweise 1, 2, 3 oder 4, weitere Aminosäuresubstitution(en) auf, wobei diese vorzugsweise ausgewählt sind aus: 62S, 149I, 156G, 156Q, 166M, 166Q, 166A, 172P, 172G oder 217M. Derartige Proteasen sind beispielsweise als Mutanten 7-12 in Beispiel 1 offenbart und damit Gegenstand der Erfindung. Besonders bevorzugt sind solche Proteasen, die an der Position, die Position 271 entspricht, eine Aminosäuresubstitution, insbesondere die Aminosäuresubstitution 271E; und an den Positionen, die den Positionen 9, 130, 144 und 252 entsprechen, die Aminosäuresubstitutionen 9T, 130D, 144K und 252T; und an einer oder mehreren der Positionen, die den Positionen 62, 166 oder 217, entsprechen, mindestens eine, beispielsweise 1, 2 oder 3, weitere Aminosäuresubstitution(en) auf, wobei diese vorzugsweise ausgewählt sind aus: 62S, 166Q und 217M.

In weiteren Ausführungsformen weist die Protease Aminosäuresubstitutionen an den Positionen auf (in der Zählung gemäß SEQ ID NO:1):
9 + 271 + 29 + 48 + 101
9 + 271 + 29 + 48 + 130
9 + 271 + 29 + 48 + 131
9 + 271 + 29 + 48 + 133
9 + 271 + 29 + 48 + 144
9 + 271 + 29 + 48 + 224
9 + 271 + 29 + 48 + 252
9 + 271 + 29 + 101 + 130
9 + 271 + 29 + 101 + 131
9 + 271 + 29 + 101 + 133
9 + 271 + 29 + 101 + 144
9 + 271 + 29 + 101 +224
9 + 271 + 29 + 101 +252
9 + 271 + 29 + 130 + 131
9 + 271 + 29 + 130 + 133
9 + 271 + 29 + 130 + 144
9 + 271 + 29 + 130 + 224
9 + 271 + 29 + 130 + 252
9 + 271 + 29 + 131 + 133
9 + 271 + 29 + 131 + 144
9 + 271 + 29 + 131 +224
9 + 271 + 29 + 131 + 252
9 + 271 + 29 + 133 + 144
9 + 271 + 29 + 133 + 224
9 + 271 + 29 + 133 + 252
9 + 271 + 29 + 144 + 224
9 + 271 + 29 + 144 + 252
9 + 271 + 29 + 224 + 252
9 + 271 + 48 + 101 + 130
9 + 271 + 48 + 101 + 131
9 + 271 + 48 + 101 + 133
9 + 271 + 48 + 101 + 144
9 + 271 + 48 + 101 + 224
9 + 271 + 48 + 101 + 252
9 + 271 + 48 + 130 + 131
9 + 271 + 48 + 130 + 133
9 + 271 + 48 + 130 + 144
9 + 271 + 48 + 130 + 224
9 + 271 + 48 + 130 + 252
9 + 271 + 48 + 131 + 133
9 + 271 + 48 + 131 + 144
9 + 271 + 48 + 131 + 224
9 + 271 + 48 + 131 + 252
9 + 271 + 48 + 133 + 144
9 + 271 + 48 + 133 + 224
9 + 271 + 48 + 133 + 252
9 + 271 + 48 + 144 + 224
9 + 271 + 48 + 144 + 252
9 + 271 + 48 + 224 + 252
9 + 271 + 101 + 130 + 131
9 + 271 + 101 + 130 + 133
9 + 271 + 101 + 130 + 144
9 + 271 + 101 + 130 + 224
9 + 271 + 101 + 130 + 252
9 + 271 + 101 + 131 + 133
9 + 271 + 101 + 131 + 144
9 + 271 + 101 + 131 + 224
9 + 271 + 101 + 131 + 252
9 + 271 + 101 + 133 + 144
9 + 271 + 101 + 133 + 224
9 + 271 + 101 + 133 + 252
9 + 271 + 101 + 144 + 224
9 + 271 + 101 + 144 + 252
9 + 271 + 101 + 224 + 252
9 + 271 + 130 + 131 + 133
9 + 271 + 130 + 131 + 144
9 + 271 + 130 + 131 + 224
9 + 271 + 130 + 131 + 252
9 + 271 + 130 + 133 + 144
9 + 271 + 130 + 133 + 224
9 + 271 + 130 + 133 + 252
9 + 271 + 130 + 144 + 224
9 + 271 + 130 + 144 + 252
9 + 271 + 130 + 224 + 252
9 + 271 + 131 + 133 + 144
9 + 271 + 131 + 133 + 224
9 + 271 + 131 + 133 + 252
9 + 271 + 131 + 144 + 224
9 + 271 + 131 + 144 + 252
9 + 271 + 131 + 224 + 252
9 + 271 + 133 + 144 + 224
9 + 271 + 133 + 144 + 252
9 + 271 + 133 + 224 + 252
9 + 271 + 144 + 224 + 252
9 + 271 + 29 + 48 + 101 + 130
9 + 271 + 29 + 48 + 101 + 131
9 + 271 + 29 + 48 + 101 + 133
9 + 271 + 29 + 48 + 101 + 144
9 + 271 + 29 +48 + 101 + 224
9 + 271 + 29 +48 + 101 + 252
9 + 271 + 29 + 48 + 130 + 131
9 + 271 + 29 + 48 + 130 + 133
9 + 271 + 29 + 48 + 130 + 144
9 + 271 + 29 + 48 + 130 + 224
9 + 271 + 29 + 48 + 130 + 252
9 + 271 + 29 + 48 + 131 + 133
9 + 271 + 29 + 48 + 131 + 144
9 + 271 + 29 + 48 + 131 + 224
9 + 271 + 29 + 48 + 131 + 252
9 + 271 + 29 + 48 + 133 + 144
9 + 271 + 29 + 48 + 133 + 224
9 + 271 + 29 + 48 + 133 + 252
9 + 271 + 29 + 101 + 130 + 131
9 + 271 + 29 + 101 + 130 + 133
9 + 271 + 29 + 101 + 130 + 144
9 + 271 + 29 + 101 + 130 + 224
9 + 271 + 29 + 101 + 130 + 252
9 + 271 + 29 + 101 + 131 + 133
9 + 271 + 29 + 101 + 131 + 144
9 + 271 + 29 + 101 + 131 + 224
9 + 271 + 29 + 101 + 131 + 252
9 + 271 + 29 + 101 + 133 + 144
9 + 271 + 29 + 101 + 133 + 224
9 + 271 + 29 + 101 + 133 + 252
9 + 271 + 29 + 101 + 144 + 224
9 + 271 + 29 + 101 + 144 + 252
9 + 271 + 29 + 101 + 224 + 252
9 + 271 + 29 + 130 + 131 + 133
9 + 271 + 29 + 130 + 131 + 144
9 + 271 + 29 + 130 + 131 + 224
9 + 271 + 29 + 130 + 131 + 252
9 + 271 + 29 + 130 + 133 + 144
9 + 271 + 29 + 130 + 133 + 224
9 + 271 + 29 + 130 + 133 + 252
9 + 271 + 29 + 130 + 144 + 224
9 + 271 + 29 + 130 + 144 + 252
9 + 271 + 29 + 130 + 224 + 252
9 + 271 + 29 + 131 + 133 + 144
9 + 271 + 29 + 131 + 133 + 224
9 + 271 + 29 + 131 + 133 + 252
9 + 271 + 29 + 131 + 144 + 224
9 + 271 + 29 + 131 + 144 + 252
9 + 271 + 29 + 131 + 224 + 252
9 + 271 + 29 + 133 + 144 + 224
9 + 271 + 29 + 133 + 144 + 252
9 + 271 + 29 + 133 + 224 + 252
9 + 271 + 29 + 144 + 224 + 252
9 + 271 + 48 + 101 + 130 + 131
9 + 271 + 48 + 101 + 130 + 133
9 + 271 + 48 + 101 + 130 + 144
9 + 271 + 48 + 101 + 130 + 224
9 + 271 + 48 + 101 + 130 + 252
9 + 271 + 48 + 101 + 131 + 133
9 + 271 + 48 + 101 + 131 + 144
9 + 271 + 48 + 101 + 131 + 224
9 + 271 + 48 + 101 + 131 + 252
9 + 271 + 48 + 101 + 133 + 144
9 + 271 + 48 + 101 + 133 + 224
9 + 271 + 48 + 101 + 133 + 252
9 + 271 + 48 + 101 + 144 + 224
9 + 271 + 48 + 101 + 144 + 252
9 + 271 + 48 + 101 + 224 + 252
9 + 271 + 48 + 130 + 131 + 133
9 + 271 + 48 + 130 + 131 + 144
9 + 271 + 48 + 130 + 131 + 224
9 + 271 + 48 + 130 + 131 + 252
9 + 271 + 48 + 130 + 133 + 144
9 + 271 + 48 + 130 + 133 + 224
9 + 271 + 48 + 130 + 133 + 252
9 + 271 + 48 + 130 + 144 + 224
9 + 271 + 48 + 130 + 144 + 252
9 + 271 + 48 + 130 + 224 + 252
9 + 271 + 48 + 131 + 133 + 144
9 + 271 + 48 + 131 + 133 + 224
9 + 271 + 48 + 131 + 133 + 252
9 + 271 + 48 + 131 + 144 + 224
9 + 271 + 48 + 131 + 144 + 252
9 + 271 + 48 + 131 + 224 + 252
9 + 271 + 48 + 133 + 144 + 224
9 + 271 + 48 + 133 + 144 + 252
9 + 271 + 48 + 133 + 224 + 252
9 + 271 + 48 + 144 + 224 + 252
9 + 271 + 101 + 130 + 131 + 133
9 + 271 + 101 + 130 + 131 + 144
9 + 271 + 101 + 130 + 131 + 224
9 + 271 + 101 + 130 + 131 + 252
9 + 271 + 101 + 130 + 133 + 144
9 + 271 + 101 + 130 + 133 + 224
9 + 271 + 101 + 130 + 133 + 252
9 + 271 + 101 + 130 + 144 + 224
9 + 271 + 101 + 130 + 144 + 252
9 + 271 + 101 + 130 + 224 + 252
9 + 271 + 101 + 131 + 133 + 144
9 + 271 + 101 + 131 + 133 + 224
9 + 271 + 101 + 131 + 133 + 252
9 + 271 + 101 + 131 + 144 + 224
9 + 271 + 101 + 131 + 144 + 252
9 + 271 + 101 + 131 + 224 + 252
9 + 271 + 101 + 133 + 144 + 224
9 + 271 + 101 + 133 + 144 + 252
9 + 271 + 101 + 133 + 224 + 252
9 + 271 + 101 + 144 + 224 + 252
9 + 271 + 130 + 131 + 133 + 144
9 + 271 + 130 + 131 + 133 + 224
9 + 271 + 130 + 131 + 133 + 252
9 + 271 + 130 + 131 + 144 + 224
9 + 271 + 130 + 131 + 144 + 252
9 + 271 + 130 + 131 + 224 + 252
9 + 271 + 130 + 133 + 144 + 224
9 + 271 + 130 + 133 + 144 + 252
9 + 271 + 130 + 133 + 224 + 252
9 + 271 + 130 + 144 + 224 + 252
9 + 271 + 131 + 133+ 144 + 224
9 + 271 + 131 + 133+ 144 + 252
9 + 271 + 131 + 133 + 224 + 252
9 + 271 + 131 + 144 + 224 + 252
9 + 271 + 133 + 144 + 224 + 252

In verschiedenen Ausführungsformen weisen die vorgenannten Varianten keine weiteren Substitutionen auf oder nur eine oder mehrere zusätzliche Substitutionen in den Positionen aus der Gruppe der Positionen 29, 48, 101, 130, 131, 133, 144, 224 und 252, sofern diese vorstehend noch nicht genannt sind. In weiteren Ausführungsformen, insbesondere in allen vorstehend beschriebenen Ausführungsformen, weist die erfindungsgemäße Protease mindestens eine, beispielsweise 1, 2 oder 3 zusätzliche Aminosäuresubstitutionen an den Positionen auf, die den Positionen 62, 166 oder 217, bezogen auf die Nummerierung gemäß SEQ ID NO: 1, entsprechen, auf. In verschiedenen Ausführungsform ist diese zusätzliche Aminosäuresubstitution eine Substitution in Position 217. In verschiedenen anderen Ausführungsformen ist diese zusätzliche Substitution eine in Position 62 und/oder 166. Die vorstehend beschriebene Aminosäuresubstitution kann vorzugsweise ausgewählt sein aus: 62S, 166Q und 217M.

Weitere Ausführungsformen der Erfindung betreffen Proteasevarianten, die Aminosäuresubstitutionen an den folgenden Positionen aufweisen (in der Zählung gemäß SEQ ID NO:1):
9 + 130 + 144 + 252 + 271 + 62
9 + 130 + 144 + 252 + 271 + 149
9 + 130 + 144 + 252 + 271 + 156
9 + 130 + 144 + 252 + 271 + 166
9 + 130 + 144 + 252 + 271 + 172
9 + 130 + 144 + 252 + 271 + 217
9 + 130 + 144 + 252 + 271 + 62 + 149
9 + 130 + 144 + 252 + 271 + 62 + 156
9 + 130 + 144 + 252 + 271 + 62 + 166
9 + 130 + 144 + 252 + 271 + 62 + 172
9 + 130 + 144 + 252 + 271 + 62 + 217
9 + 130 + 144 + 252 + 271 + 149 + 156
9 + 130 + 144 + 252 + 271 + 149 + 166
9 + 130 + 144 + 252 + 271 + 149 + 172
9 + 130 + 144 + 252 + 271 + 149 + 217
9 + 130 + 144 + 252 + 271 + 156 + 166
9 + 130 + 144 + 252 + 271 + 156 + 172
9 + 130 + 144 + 252 + 271 + 156 + 217
9 + 130 + 144 + 252 + 271 + 166+ 172
9 + 130 + 144 + 252 + 271 + 166 + 217
9 + 130 + 144 + 252 + 271 + 172 + 217
9 + 130 + 133 + 144 + 252 + 271 + 62
9 + 130 + 133 + 144 + 252 + 271 + 149
9 + 130 + 133 + 144 + 252 + 271 + 156
9 + 130 + 133 + 144 + 252 + 271 + 166
9 + 130 + 133 + 144 + 252 + 271 + 172
9 + 130 + 133 + 144 + 252 + 271 + 217
9 + 130 + 133 + 144 + 252 + 271 + 62 + 149
9 + 130 + 133 + 144 + 252 + 271 + 62 + 156
9 + 130 + 133 + 144 + 252 + 271 + 62 + 166
9 + 130 + 133 + 144 + 252 + 271 + 62 + 172
9 + 130 + 133 + 144 + 252 + 271 + 62 + 217
9 + 130 + 133 + 144 + 252 + 271 + 149 + 156
9 + 130 + 133 + 144 + 252 + 271 + 149 + 166
9 + 130 + 133 + 144 + 252 + 271 + 149 + 172
9 + 130 + 133 + 144 + 252 + 271 + 149 + 217
9 + 130 + 133 + 144 + 252 + 271 + 156 + 166
9 + 130 + 133 + 144 + 252 + 271 + 156 + 172
9 + 130 + 133 + 144 + 252 + 271 + 156 + 217
9 + 130 + 133 + 144 + 252 + 271 + 166 + 172
9 + 130 + 133 + 144 + 252 + 271 + 166 + 217
9 + 130 + 133 + 144 + 252 + 271 + 172 + 217

In verschiedenen Ausführungsformen weisen die vorgenannten Varianten keine weiteren Substitutionen auf oder nur eine oder mehrere zusätzliche Substitutionen in den Positionen aus der Gruppe der Positionen 62, 133, 149, 156, 166, 172 und 217 sofern diese vorstehend noch nicht genannt sind.

In allen vorgenannten Varianten sind die entsprechenden Austausche insbesondere die oben erwähnten, d.h. 9T, 9H, 9S, 9A, 29G, 48V, 101E, 130D, 130S, 130H, 131D, 131N, 131S, 131K, 133K, 133R, 133Y, 144K, 144L, 144A, 224A, 224T, 252S, 252T und/oder 271E. Ganz besonders bevorzugt sind 9T, 130D, 133R/K, 144K, 252T/S und/oder 271E, insbesondere 9T, 130D, 133R/K, 144K, 252T und 271E oder 9T, 130D, 133K, 144K, 252T und 271E. Dementsprechend sind insbesondere die oben genannten Varianten, in denen die vorgenannten Substitutionen vorkommen bevorzugt. Weiter bevorzugt ist eine Variante, die die Substitutionen 9T, 130D, 144K, 252T und 271E aufweist, sowie optional auch 133R/K und/oder 217M.

In bevorzugten Ausführungsformen der erfindungsgemäßen Protease, d.h. insbesondere den oben aufgelisteten Varianten, weist die Protease an den Positionen, die den Positionen 62, 149, 156, 166, 172 und 217 entsprechen, Aminosäuresubstitutionen auf, wobei diese vorzugsweise ausgewählt sind aus: 62S, 149I, 156G, 156Q, 166M, 166Q, 166A, 172P, 172G oder 217M.

Derartige Proteasen sind beispielsweise als Mutanten in Beispiel 1 offenbart und damit Gegenstand der Erfindung. Besonders bevorzugt sind solche Proteasen, die an der Position, die Position 271 entspricht, eine Aminosäuresubstitution, insbesondere die Aminosäuresubstitution 271E; und an den Positionen, die den Positionen 9, 130, 144 und 252 entsprechen, die Aminosäuresubstitutionen 9T, 130D, 144K und 252T; und an einer oder mehreren der Positionen, die den Positionen 62, 166 oder 217, entsprechen, mindestens eine, beispielsweise 1, 2 oder 3, weitere Aminosäuresubstitution(en) auf, wobei diese vorzugsweise ausgewählt sind aus: 62S, 166Q und 217M.

In weiteren Ausführungsformen, insbesondere in allen vorstehend beschriebenen Ausführungsformen, weist die erfindungsgemäße Protease eine zusätzliche Aminosäuresubstitution an der Position, die der Position 216, bezogen auf die Nummerierung gemäß SEQ ID NO:1, entspricht, auf. Diese Aminosäuresubstitution kann die Aminosäuresubstitution S216C sein. In verschiedenen bevorzugten Ausführungsformen betrifft die hierin beschriebene Anmeldung aber auch Proteasevarianten die nicht die Substitution 216C aufweisen, insbesondere keine Substitution in der Position 216 in der Zählung gemäß SEQ ID NO: 1 aufweisen.

Ferner enthält die erfindungsgemäße Protease in verschiedenen Ausführungsformen mindestens eine Aminosäuresubstitution, die aus der Gruppe bestehend aus A29G, A48V, D101E, N130D, N130S, N130H, G131S, G131N, G131D, G131K, T133K, T133R, T133Y, N144K, N144L, N144A, S224A, S224T oder N252S, jeweils bezogen auf die Nummerierung gemäß SEQ ID NO:1, ausgewählt ist. In noch weiter bevorzugten Ausführungsformen enthält die erfindungsgemäße Protease die Aminosäuresubstitutionen (1) P9T, P9H, P9S oder P9A und (2) Q271E und optional auch (3) S216C sowie zusätzlich eine der folgenden Aminosäuresubstitutionsvarianten: (i) N130D, G131S, T133Y und S224A; (ii) N130S, G131S, T133Y, N144L und S224A; (iii) N130D, G131N, T133K und N144K; (iv) N130H, G131N, N144K und S224A; (v) A29G, N130D, G131N und T133R; (vi) A29G, D101E, N130S, G131S, S224T und N252S; (vii) N130H, G131S und S224A; (viii) N130D, G131S, T133K und S224A; (ix) A29G, D101E, N130D, G131K und S224A; (x) N130D, G131N T133K, N144L und N252S; (xi) G131S, N144K und S224T; (xii) D101E, N130D, G131S, T133Y, N144A und S224A; (xiii) A29G, D101E, N130S, S224T und N252S; (xiv) A48V, G131S, T133R und S224A; oder (xv) G131D, T133R und S224A, wobei die Nummerierung jeweils bezogen ist auf die Nummerierung gemäß SEQ ID NO: 1.

In einer weiteren Ausführungsform weist die erfindungsgemäße Protease eine Aminosäuresequenz gemäß SEQ ID Nos. 3-17 oder 19-23 oder 20-23 auf.

Die erfindungsgemäßen Proteasen verfügen über eine verbesserte Lagerstabilität. Sie verfügen über eine erhöhte Stabilität in Wasch- oder Reinigungsmitteln im Vergleich zu einer Referenz-Mutations-Variante der Protease (SEQ ID NO:18 und/oder SEQ ID NO:2 und/oder SEQ ID NO:19), insbesondere bei einer Lagerung von 3 oder mehr Tagen, 4 oder mehr Tagen, 7 oder mehr Tagen, 10 oder mehr Tagen, 12 oder mehr Tagen, 14 oder mehr Tagen, 21 oder mehr Tagen oder 28 oder mehr Tagen. Solche stabilitätsverbesserten Proteasen ermöglichen auch nach längerer Lagerzeit gute Waschergebnisse an proteolytisch-sensitiven Anschmutzungen in verschiedenen Temperaturbereichen, insbesondere einem Temperaturbereich von 20°C bis 40°C.

Die erfindungsgemäßen Proteasen können zusätzlich zur erhöhten Lagerstabilität auch über eine erhöhte katalytische Aktivität in Wasch- oder Reinigungsmitteln verfügen. In vielfältigen Ausführungsformen können die erfindungsgemäßen Proteasen eine proteolytische Aktivität besitzen, die bezogen den Wildtyp (SEQ ID NO:1) mindestens 101%, vorzugsweise mindestens 102% oder mehr beträgt. In verschiedenen Ausführungsformen besitzen die erfindungsgemäßen Proteasen eine proteolytische Aktivität, die bezogen auf eine Referenz-Mutations-Variante der Protease (SEQ ID NO:1 und/ SEQ ID NO:2 und/oder SEQ ID NO:19) mindestens 110%, mindestens 115%, mindestens 120%, mindestens 125%, mindestens 130%, mindestens 135%, mindestens 140%, mindestens 145%, mindestens 150%, mindestens 155% oder mindestens 160% beträgt. Solche leistungsverbesserten Proteasen ermöglichen verbesserte Waschergebnisse an proteolytisch-sensitiven Anschmutzungen in verschiedenen Temperaturbereichen, insbesondere einem Temperaturbereich von 20°C bis 40°C.

Die erfindungsgemäßen Proteasen weisen enzymatische Aktivität auf, das heißt, sie sind zur Hydrolyse von Peptiden und Proteinen befähigt, insbesondere in einem Wasch- oder Reinigungsmittel. Eine erfindungsgemäße Protease ist daher ein Enzym, welches die Hydrolyse von Amid/Peptidbindungen in Protein/Peptid-Substraten katalysiert und dadurch in der Lage ist, Proteine oder Peptide zu spalten. Ferner handelt es sich bei einer erfindungsgemäßen Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen der Erfindung ist die Protease ein frei vorliegendes Enzym. Dies bedeutet, dass die Protease mit allen Komponenten eines Mittels direkt agieren kann und, falls es sich bei dem Mittel um ein Flüssigmittel handelt, dass die Protease direkt mit dem Lösungsmittel des Mittels (z.B. Wasser) in Kontakt steht. In anderen Ausführungsformen kann ein Mittel Proteasen enthalten, die einen Interaktionskomplex mit anderen Molekülen bilden oder die eine "Umhüllung" enthalten. Hierbei kann ein einzelnes oder mehrere Protease Moleküle durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von *Bacillus pumilus* oder *Bacillus subtilis,* die die erfindungsgemäßen Proteasen exprimieren, oder Zellkulturüberstände solcher Zellen enthalten.

In einer weiteren Ausführungsform der Erfindung umfasst die Protease eine Aminosäuresequenz, die zu der in SEQ ID NO:1 angegebenen Aminosäuresequenz über deren Gesamtlänge zu mindestens 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und jeweils bezogen auf die Nummerierung gemäß SEQ ID NO:1 die oben angegebenen Aminosäuresubstitutionen aufweist. Im Zusammenhang mit der vorliegenden Erfindung bedeutet das Merkmal, dass eine Protease die angegebenen Substitutionen aufweist, dass sie an der jeweiligen Position eine (der angegebenen) Substitution(en) enthält, d.h. zumindest die angegebenen Positionen nicht anderweitig mutiert oder, beispielsweise durch Fragmentierung der Protease, deletiert sind. In verschiedenen Ausführungsformen weist sie Aminosäuresubstitutionen (a) an den Positionen, die den Positionen 9 und 271, entsprechen, auf, vorzugsweise derart, dass die Aminosäuresubstitutionen P9T, P9H, P9S oder P9A und Q271E sind und weist (b) an mindestens drei der Positionen, die den Positionen 29, 48, 101, 130, 131, 133, 144, 224 oder 252 in der Zählung gemäß SEQ ID NO:1 entsprechen, drei oder mehrere der Aminosäuresubstitutionen 29G, 48V, 101E, 130D, 130S, 130H, 131S, 131N, 131D, 131K, 133K, 133R, 133Y, 144K, 144L, 144A, 224A, 224T oder 252S auf. Die Aminosäuresequenzen derartiger Proteasen, die erfindungsgemäß bevorzugt sind, sind in SEQ ID Nos: 3-17 und 19-23 angegeben. In verschiedenen Ausführungsformen weisen die hierin beschriebenen Proteasen mit Ausnahme der explizit erwähnten Substitutionen die Sequenz von SEQ ID NO:1 auf, d.h. sind abgesehen von den substituierten Positionen 100% identisch zu der Sequenz gemäß SEQ ID NO:1.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul et al. (1990): "Basic local alignment search tool", J. Mol. Biol. 215:403-410, und Altschul et al. (1997): "Gapped BLAST and PSI-BLAST: a new generation of protein database search programs", Nucleic Acids Res. 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): "Multiple sequence alignment with the Clustal series of programs", Nucleic Acid Res. 31 :3497-3500), T-Coffee (vgl. beispielsweise Notredame et al. (2000): "T-Coffee: A novel method for multiple sequence alignments", J. Mol. Biol. 302:205-217) oder Programme, die auf diesen Programmen beziehungsweise Algorithmen basieren. Ferner möglich sind Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert. Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäureaustausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO: 1 in einem wie oben definierten Alignment zugeordnet ist.

In einer weiteren Ausführungsform der Erfindung ist die Protease dadurch gekennzeichnet, dass ihre Reinigungsleistung (nach Lagerung, z.B. über 4 Wochen) gegenüber derjenigen einer Protease, die eine Aminosäuresequenz umfasst, die der in SEQ ID NO:18 und/oder SEQ ID NO:2 und/oder SEQ ID NO:19 angegebenen Aminosäuresequenz entspricht, nicht signifikant verringert ist, d.h. mindestens 80% der Referenzwaschleistung besitzt, vorzugsweise mindestens 100%, noch bevorzugter mindestens 110% oder mehr. Die Reinigungsleistung kann in einem Waschsystem bestimmt werden, das ein Waschmittel in einer Dosierung zwischen 4,5 und 7,0 Gramm pro Liter Waschflotte sowie die Protease enthält, wobei die zu vergleichenden Proteasen konzentrationsgleich (bezogen auf aktives Protein) eingesetzt sind und die Reinigungsleistung gegenüber einer Anschmutzung auf Baumwolle bestimmt wird durch Messung des Reinigungsgrades der gewaschenen Textilien. Beispielsweise kann der Waschvorgang für 60 Minuten bei einer Temperatur von 40°C erfolgen und das Wasser eine Wasserhärte zwischen 15,5 und 16,5° (deutsche Härte) aufweisen. Die Konzentration der Protease in dem für dieses Waschsystem bestimmten Waschmittel beträgt 0,001 bis 0,1 Gew.-%, vorzugsweise 0,01 bis 0,06 Gew.-%, bezogen auf aktives, gereinigtes Protein.

Ein flüssiges Referenzwaschmittel für ein solches Waschsystem kann wie folgt zusammengesetzt sein (alle Angaben in Gewichts-Prozent): 4,4% Alkylbenzolsulfonsäure, 5,6% weitere anionische Tenside, 2,4% C12-C18 Na-Salze von Fettsäuren (Seifen), 4,4% nicht-ionische Tenside, 0,2% Phosphonate, 1,4% Zitronensäure, 0,95% NaOH, 0,01% Entschäumer, 2% Glycerin, 0,08% Konservierungsstoffe, 1% Ethanol, Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird gewaschen in einem pH-Wertebereich zwischen pH 7 und pH 10,5, bevorzugt zwischen pH 7,5 und pH 8,5.

Im Rahmen der Erfindung erfolgt die Bestimmung der Reinigungsleistung beispielsweise bei 20°C oder 40°C unter Verwendung eines flüssigen Waschmittels wie vorstehend angegeben, wobei der Waschvorgang vorzugsweise für 60 Minuten bei 600 rpm erfolgt.

Der Weißegrad, d.h. die Aufhellung der Anschmutzungen, als Maß für die Reinigungsleistung wird mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Durch den aktivitätsgleichen Einsatz der jeweiligen Protease wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also beispielsweise die Reinigungsleistung an bestimmten Anschmutzungen, verglichen werden. Generell gilt, dass eine niedrige spezifische Aktivität durch Zugabe einer größeren Proteinmenge ausgeglichen werden kann.

Ansonsten sind Verfahren zur Bestimmung der Proteaseaktivität dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Alternativ kann die Proteaseaktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20s bis 60s. Die Proteaseaktivität wird üblicherweise in Protease-Einheiten (PE) angegeben. Geeignete Proteaseaktivitäten betragen beispielsweise 2,25, 5 oder 10 PE pro ml Waschflotte. Die Proteaseaktivität ist jedoch nicht gleich Null.

Ein alternativer Test zur Feststellung der proteolytischen Aktivität der erfindungsgemäßen Proteasen ist ein optisches Messverfahren, bevorzugt ein photometrisches Verfahren. Der hierfür geeignete Test umfasst die Protease-abhängige Spaltung des Substratproteins Casein. Dieses wird durch die Protease in eine Vielzahl kleinerer Teilprodukte gespalten. Die Gesamtheit dieser Teilprodukte weist eine erhöhte Absorption bei 290 nm gegenüber nicht gespaltenem Casein auf, wobei diese erhöhte Absorption unter Verwendung eines Photometers ermittelt werden und somit ein Rückschluss auf die enzymatische Aktivität der Protease gezogen werden kann.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (vgl. M. Bender et al., J. Am. Chem. Soc. 88, 24 (1966), S. 5890-5913) erfolgen.

Zusätzlich zu den vorstehend erläuterten Aminosäureveränderungen können erfindungsgemäße Proteasen weitere Aminosäureveränderungen, insbesondere Aminosäure-Substitutionen, -Insertionen oder -Deletionen, aufweisen. Solche Proteasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden.

Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität der Protease erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierter Proteasen, zum Beispiel hinsichtlich ihrer Stabilität bei Lagerung, kann daher im Rahmen der Erfindung zusätzlich weiterentwickelt sein.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt oder vor der entsprechenden Position ein Δ angegeben. Beispielsweise beschreibt P9T die Substitution von Prolin an Position 9 durch Threonin, P9TH die Insertion von Histidin nach der Aminosäure Threonin an Position 9 und P9* oder ΔP9 die Deletion von Prolin an Position 9. Diese Nomenklatur ist dem Fachmann auf dem Gebiet der Enzymtechnologie bekannt.

Ein weiterer Gegenstand der Erfindung ist daher eine Protease, die dadurch gekennzeichnet ist, dass sie aus einer Protease wie vorstehend beschrieben als Ausgangsmolekül erhältlich ist durch ein- oder mehrfache konservative Aminosäuresubstitution, wobei die Protease in der Zählung gemäß SEQ ID NO:1 die vorstehend beschriebenen Aminosäuresubstitutionen aufweist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Alternativ oder ergänzend ist die Protease dadurch gekennzeichnet, dass sie aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 200, 210, 220, 230, 240, 250, 260, 261, 262, 263, 264, 265, 266, 267, 268, 269, 270, 271, 272, 273 oder 274 oder 275 zusammenhängenden Aminosäuren mit dem Ausgangsmolekül übereinstimmt, wobei die im Ausgangsmolekül eventuell enthaltene(n) oben beschriebenen Aminosäuresubstitution(en) gemäß der Erfindung, d.h. die Substitutionen an den Positionen, die den Positionen 9, 29, 48, 101, 130, 131, 133, 144, 216, 224, 252 und 271 in SEQ ID NO:1 entsprechen, sowie optional die Aminosäuresubstitution 166M, 166Q oder 166A an der Position, die der Position 166 entspricht, und/oder mindestens eine weitere Aminosäuresubstitution an mindestens einer der Positionen, die den Positionen 62, 133, 149, 156, 172 oder 217 entsprechen, noch vorhanden sind.

So ist es beispielsweise möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms, d.h. in einer bevorzugten Ausführungsform beträgt die proteolytische Aktivität mindestens 80%, vorzugsweise mindestens 90% der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Alternativ oder ergänzend ist die Protease dadurch gekennzeichnet, dass sie aus einer erfindungsgemäßen Protease als Ausgangsmolekül erhältlich ist durch ein- oder mehrfache konservative Aminosäuresubstitution, wobei die Protease (i) die Aminosäuresubstitutionen P9T, P9H, P9S oder P9A und Q271E an den Positionen, die den Positionen 9 und 271 gemäß SEQ ID NO:1 entsprechen, und (ii) mindestens drei der Aminosäuresubstitutionen A29G, A48V, D101E, N130D, N130S, N130H, G131S, G131N, G131D, G131K, T133K, T133R, T133Y, N144K, N144L, N144A, S224A, S224T oder N252S an den Positionen, die den Positionen 29, 48, 101, 130, 131, 133, 144, 224 und 252 gemäß SEQ ID NO:1 entsprechen, sowie optional die Aminosäuresubstitution 166M, 166Q oder 166A an der Position, die der Position 166 entspricht, und/oder mindestens eine weitere Aminosäuresubstitution an mindestens einer der Positionen, die den Positionen 62, 133, 149, 156, 172 oder 217 entsprechen, aufweist.

In weiteren Ausführungsformen ist die Protease dadurch gekennzeichnet, dass sie aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 200, 210, 220, 230, 240, 250, 260, 261, 262, 263, 264, 265, 266, 267, 268, 269, 270, 271, 272, 273 oder 274 oder 275 zusammenhängende Aminosäuren mit dem Ausgangsmolekül übereinstimmt, wobei die Protease (i) die Aminosäuresubstitutionen P9T, P9H, P9S oder P9A und Q271E an den Positionen, die den Positionen 9 und 271 gemäß SEQ ID NO:1 entsprechen, und (ii) mindestens drei der Aminosäuresubstitutionen A29G, A48V, D101E, N130D, N130S, N130H, G131S, G131N, G131D, G131K, T133K, T133R, T133Y, N144K, N144L, N144A, S224A, S224T oder N252S an den Positionen, die den Positionen 29, 48, 101, 130, 131, 133, 144, 224 und 252 gemäß SEQ ID NO:1 entsprechen, sowie optional die Aminosäuresubstitution 166M, 166Q oder 166A an der Position, die der Position 166 entspricht, und/oder mindestens eine weitere Aminosäuresubstitution an mindestens einer der Positionen, die den Positionen 62, 133, 149, 156, 172 oder 217 entsprechen, umfasst.

Die weiteren Aminosäurepositionen werden hierbei durch ein Alignment der Aminosäuresequenz einer erfindungsgemäßen Protease mit der Aminosäuresequenz der Protease aus *Bacillus pumilus,* wie sie in SEQ ID NO:1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Protease eine höhere Zahl von Aminosäureresten umfasst als die Protease aus *Bacillus pumilus* gemäß SEQ ID NO:1. Ausgehend von den genannten Positionen in der Aminosäuresequenz der Protease aus *Bacillus pumilus* sind die Veränderungspositionen in einer erfindungsgemäßen Protease diejenigen, die eben diesen Positionen in einem Alignment zugeordnet sind.

Vorteilhafte Positionen für Sequenzveränderungen, insbesondere Substitutionen, der Protease aus *Bacillus pumilus,* die übertragen auf homologe Positionen der erfindungsgemäßen Proteasen bevorzugt von Bedeutung sind und der Protease vorteilhafte funktionelle Eigenschaften verleihen, sind demnach die Positionen, die in einem Alignment den Positionen 9, 29, 48, 62, 101, 130, 131, 133, 144, 149, 156, 166, 172, 216, 217, 224, 252 und 271 in SEQ ID NO:1 entsprechen, d.h. in der Zählung gemäß SEQ ID NO:1. An den genannten Positionen liegen in dem Wildtypmolekül der Protease aus *Bacillus pumilus* folgende Aminosäurereste: P9, A29, A48, Q62, D101, N130, G131, T133, N144, V149, S156, G166, D172, S216, Y217, S224, N252 und Q271.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Proteasen auf die gleiche Weise verändert werden und beobachtet wird, ob bei beiden die enzymatische Aktivität auf gleiche Weise verändert wird. Geht beispielsweise ein Aminosäureaustausch in einer bestimmten Position der Protease aus *Bacillus pumilus* gemäß SEQ ID NO:1 mit einer Veränderung eines enzymatischen Parameters einher, beispielsweise mit der Erhöhung des K_{M}-Wertes, und wird eine entsprechende Veränderung des enzymatischen Parameters, beispielsweise also ebenfalls eine Erhöhung des K_{M}-Wertes, in einer erfindungsgemäßen Protease-Variante beobachtet, deren Aminosäureaustausch durch dieselbe eingeführte Aminosäure erreicht wurde, so ist hierin eine Bestätigung der korrekten Zuordnung zu sehen.

Alle genannten Sachverhalte sind auch auf die erfindungsgemäßen Verfahren zur Herstellung einer Protease anwendbar. Demnach umfasst ein erfindungsgemäßes Verfahren ferner einen oder mehrere der folgenden Verfahrensschritte:
a) Einbringen einer ein- oder mehrfachen konservativen Aminosäuresubstitution, wobei die Protease
   (i) die Aminosäuresubstitutionen P9T, P9H, P9S oder P9A und Q271E an den Positionen, die den Positionen 9 und 271 gemäß SEQ ID NO:1 entsprechen, und
   (ii) mindestens drei der Aminosäuresubstitutionen A29G, A48V, D101E, N130D, N130S, N130H, G131S, G131N, G131D, G131K, T133K, T133R, T133Y, N144K, N144L, N144A, S224A, S224T oder N252S an den Positionen, die den Positionen 29, 48, 101, 130, 131, 133, 144, 224 und 252 gemäß SEQ ID NO:1 entsprechen, umfasst;
b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 200, 210, 220, 230, 240, 250, 260, 261, 262, 263, 264, 265, 266, 267, 268, 269, 270, 271, 272, 273 oder 274 oder 275 zusammenhängenden Aminosäuren mit dem Ausgangsmolekül übereinstimmt, wobei die Protease
   (i) die Aminosäuresubstitutionen P9T, P9H, P9S oder P9A und Q271E an den Positionen, die den Positionen 9 und 271 gemäß SEQ ID NO:1 entsprechen, und
   (ii) mindestens drei der Aminosäuresubstitutionen A29G, A48V, D101E, N130D, N130S, N130H, G131S, G131N, G131D, G131K, T133K, T133R, T133Y, N144K, N144L, N144A, S224A, S224T oder N252S an den Positionen, die den Positionen 29, 48, 101, 130, 131, 133, 144, 224 und 252 gemäß SEQ ID NO:1 entsprechen, umfasst.

In verschiedenen Ausführungsformen dieses Verfahrens umfasst dies einen oder mehrere der folgenden Schritte:
a) Einbringen einer ein- oder mehrfachen konservativen Aminosäuresubstitution in die Protease, wobei die Protease die Substitutionen 9T, 130D, 144K, 252T und 271E an den Positionen, die den Positionen 9, 130, 144, 252 und 271 entsprechen; sowie optional die Aminosäuresubstitution 166M, 166Q oder 166A an der Position, die der Position 166 entspricht, und/oder mindestens eine weitere Aminosäuresubstitution an mindestens einer der Positionen, die den Positionen 62, 133, 149, 156, 172 oder 217 entsprechen, umfasst;
b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 200, 210, 220, 230, 240, 250, 260, 261, 262, 263, 264, 265, 266, 267, 268, 269, 270, 271, 272, 273 oder 274 zusammenhängenden Aminosäuren mit dem Ausgangsmolekül übereinstimmt, wobei die Protease die Substitutionen 9T, 130D, 144K, 252T und 271E an den Positionen, die den Positionen 9, 130, 144, 252 und 271 entsprechen; sowie optional die Aminosäuresubstitution 166M, 166Q oder 166A an der Position, die der Position 166 entspricht, und/oder mindestens eine weitere Aminosäuresubstitution an mindestens einer der Positionen, die den Positionen 62, 133, 149, 156, 172 oder 217 entsprechen, umfasst.

Sämtliche Ausführungsformen gelten auch für die erfindungsgemäßen Verfahren.

In weiteren Ausgestaltungen der Erfindung ist die Protease beziehungsweise die mit einem erfindungsgemäßen Verfahren hergestellte Protease noch mindestens zu 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, oder 98,8% identisch zu der in SEQ ID NO:1 angegebenen Aminosäuresequenz über deren Gesamtlänge. Alternativ ist die Protease beziehungsweise die mit einem erfindungsgemäßen Verfahren hergestellte Protease noch mindestens zu 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, oder 98% identisch zu einer der in SEQ ID Nos:3-17 oder 19-23 angegebenen Aminosäuresequenzen über deren Gesamtlänge. Die Protease beziehungsweise die mit einem erfindungsgemäßen Verfahren hergestellte Protease weist eine Aminosäuresubstitution an den Positionen 9 und 271 und mindestens drei der Positionen, die den Positionen 29, 48, 101, 130, 131, 133, 144, 224 oder 252, jeweils bezogen auf die Nummerierung gemäß SEQ ID NO:1, entsprechen, auf. In bevorzugteren Ausführungsformen ist die Aminosäuresubstitution mindestens eine ausgewählt aus der Gruppe bestehend aus P9T, P9H, P9S, P9A, A29G, A48V, D101E, N130D, N130S, N130H, G131S, G131N, G131D, G131K, T133K, T133R, T133Y, N144K, N144L, N144A, S224A, S224T, N252S und Q271E, jeweils bezogen auf die Nummerierung gemäß SEQ ID NO:1. In weiteren Ausführungsformen umfasst die Protease weiterhin eine Substitution an der Position 216, insbesondere S216C, wobei es in verschiedenen Ausführungsformen bevorzugt sein kann, dass diese Position nicht substituiert ist. Ganz besonders bevorzugt sind die folgenden Aminosäuresubstitutionsvarianten: P9T, P9H, P9S oder P9A, insbesondere P9T, und Q271E und optional auch S216C kombiniert mit einer von (i) N130D, G131S, T133Y und S224A; (ii) N130S, G131S, T133Y, N144L und S224A; (iii) N130D, G131N, T133K und N144K; (iv) N130H, G131N, N144K und S224A; (v) A29G, N130D, G131N und T133R; (vi) A29G, D101E, N130S, G131S,

S224T und N252S; (vii) N130H, G131S und S224A; (viii) N130D, G131S, T133K und S224A; (ix) A29G, D101E, N130D, G131K und S224A; (x) N130D, G131N T133K, N144L und N252S; (xi) G131S, N144K und S224T; (xii) D101E, N130D, G131S, T133Y, N144A und S224A; (xiii) A29G, D101E, N130S, S224T und N252S; (xiv) A48V, G131S, T133R und S224A; oder (xv) G131D, T133R und S224A, wobei die Nummerierung jeweils bezogen ist auf die Nummerierung gemäß SEQ ID NO:1.

In weiteren Ausführungsformen weist die Protease beziehungsweise die mit einem erfindungsgemäßen Verfahren hergestellte Protease die Aminosäuresubstitutionen 9T, 130D, 144K, 252T und 271E an den Positionen, die den Positionen 9, 130, 144, 252 und 271 entsprechen; sowie optional die Aminosäuresubstitution 166M, 166Q oder 166A an der Position, die der Position 166 entspricht, und/oder mindestens eine weitere Aminosäuresubstitution an mindestens einer der Positionen, die den Positionen 62, 133, 149, 156, 172 oder 217 entsprechen, jeweils bezogen auf die Nummerierung gemäß SEQ ID NO: 1, auf. Beispiele hierfür sind die folgenden Aminosäuresubstitutionsvarianten: P9T und Q271E kombiniert mit einer von (i) N130D, N144K, N252T und G166M; (ii) N130D, N144K, N252T und G166Q; (iii) N130D, N144K, N252T und G166A; (iv) N130D, N144K, N252T und S156G; oder (v) N130D, N144K, N252T und Y217M, wobei die Nummerierung jeweils bezogen ist auf die Nummerierung gemäß SEQ ID NO:1 sowie die in den Beispielen beschriebenen Varianten.

Ein weiterer Gegenstand der Erfindung ist eine zuvor beschriebene Protease, die zusätzlich stabilisiert ist, insbesondere durch eine oder mehrere Mutationen, beispielsweise Substitutionen, oder durch Kopplung an ein Polymer. Eine Erhöhung der Stabilität bei der Lagerung und/oder während des Einsatzes, beispielsweise beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt.

Weitere Möglichkeiten der Stabilisierung sind beispielsweise:
- Veränderung der Bindung von Metallionen, insbesondere der Calcium-Bindungsstellen, beispielsweise durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Protease wie vorstehend beschrieben, die dadurch gekennzeichnet ist, dass sie mindestens eine chemische Modifikation aufweist. Eine Protease mit einer solchen Veränderung wird als Derivat bezeichnet, d.h. die Protease ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen kombiniert sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden. Insbesondere die gemeinsame Präparation von Proteasen mit spezifischen Inhibitoren ist diesbezüglich möglich.

Unter allen vorstehend beschriebenen Proteasen beziehungsweise Proteasevarianten und/oder Derivaten sind im Rahmen der vorliegenden Erfindung diejenigen besonders bevorzugt, deren Lagerstabilität mindestens einer derjenigen der Proteasen gemäß SEQ ID Nos: 3-17 oder 19-23 entspricht, und/oder deren Reinigungsleistung mindestens einer derjenigen der Proteasen gemäß SEQ ID Nos: 3-17 oder 19-23 entspricht, wobei die Reinigungsleistung in einem Waschsystem bestimmt wird wie vorstehend beschrieben.

Ein weiterer Gegenstand der Erfindung ist eine Nukleinsäure, die für eine erfindungsgemäße Protease codiert, sowie ein Vektor enthaltend eine solche Nukleinsäure, insbesondere ein Klonierungsvektor oder ein Expressionsvektor.

Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesen Erfindungsgegenstand mit eingeschlossen, die eine der vorstehend beschriebenen Proteasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codon(s) durch synonyme Codons ersetzt sein. Dieser Aspekt bezieht sich insbesondere auf die heterologe Expression der erfindungsgemäßen Enzyme. So besitzt jeder Organismus, beispielsweise eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press. bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zelllinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen beispielsweise solche, deren Ursprung bakterielle Plasmide, Viren oder Bakteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht menschliche Wirtszelle, die eine erfindungsgemäße Nukleinsäure oder einen erfindungsgemäßen Vektor beinhaltet, oder die eine erfindungsgemäße Protease beinhaltet, insbesondere eine, die die Protease in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Proteasen von den sie produzierenden Zellen nach deren Herstellung modifiziert werden, beispielsweise durch Anknüpfung von Zuckermolekülen, Formylierungen, Aminierungen, usw. Solche posttranslationale Modifikationen können die Protease funktionell beeinflussen.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryotische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der *Actinomycetales* besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist.

Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen, besonders bevorzugt auf solche der Gattung *Bacillus,* anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar.

In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von *Escherichia, Klebsiella, Bacillus, Staphylococcus, Corynebacterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor* und *Stenotrophomonas maltophilia.*

Die Wirtszelle kann aber auch eine eukaryontische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryotischen Zellen sind eukaryotische Zellen in der Lage, das gebildete Protein posttranslational zu modifizieren. Beispiele dafür sind Pilze wie Actinomyceten oder Hefen wie *Saccharomyces* oder *Kluyveromyces.* Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen, kann vorteilhaft sein. Ferner können sich beispielsweise thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Proteasen herzustellen. Ein weiterer Gegenstand der Erfindung ist daher ein Verfahren zur Herstellung einer Protease umfassend
a) Kultivieren einer erfindungsgemäßen Wirtszelle, und
b) Isolieren der Protease aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise der erfindungsgemäßen Proteasen. Alle Fermentationsverfahren, die auf einem entsprechenden Verfahren zur Herstellung einer erfindungsgemäßen Protease beruhen, stellen Ausführungsformen dieses Erfindungsgegenstandes dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse beziehungsweise Trockenmasse und/oder insbesondere in der Aktivität der interessierenden Protease erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Die hergestellte Protease kann aus dem Fermentationsmedium geerntet werden. Ein solches Fermentationsverfahren ist gegenüber einer Isolation der Protease aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Protease in das Fermentationsmedium sezernieren. Ohne Sekretion kann alternativ die Isolation der Protease aus der Wirtszelle, d.h. eine Aufreinigung derselben aus der Zellmasse, erfolgen, beispielsweise durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Protease herzustellen.

Ein weiterer Gegenstand der Erfindung ist ein Mittel, das dadurch gekennzeichnet ist, dass es eine erfindungsgemäße Protease wie vorstehend beschrieben enthält. Bevorzugt ist das Mittel als ein Wasch- oder Reinigungsmittel.

Zu diesem Erfindungsgegenstand zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche beziehungsweise -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmitteln im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als pulverförmige Feststoffe, in nachverdichteter Teilchenform, als homogene Lösungen oder Suspensionen vorliegen können, können neben einer erfindungsgemäßen Protease alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Persauerstoffverbindungen oder Bleichaktivatoren enthalten. Ferner können sie wassermischbare organische Lösungsmittel, weitere Enzyme, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten.

Insbesondere eine Kombination einer erfindungsgemäßen Protease mit einem oder mehreren weiteren Inhaltsstoff(en) des Mittels ist vorteilhaft, da ein solches Mittel in bevorzugten erfindungsgemäßen Ausgestaltungen eine verbesserte Reinigungsleistung durch sich ergebende Synergismen aufweist. Insbesondere durch die Kombination einer erfindungsgemäßen Protease mit einem Tensid und/oder einem Builder (Gerüststoff) und/oder einer Persauerstoffverbindung und/oder einem Bleichaktivator kann ein solcher Synergismus erreicht werden. Allerdings kann in bevorzugten Ausführungsformen das erfindungsgemäße Mittel keine Borsäure enthalten.

Vorteilhafte Inhaltsstoffe erfindungsgemäßer Mittel sind offenbart in der internationalen Patentanmeldung WO2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

Ein erfindungsgemäßes Mittel enthält die Protease vorteilhafterweise in einer Menge von 2µg bis 20mg, vorzugsweise von 5µg bis 17,5mg, besonders bevorzugt von 20µg bis 15mg und ganz besonders bevorzugt von 50µg bis 10mg pro g des Mittels. In verschiedenen Ausführungsformen beträgt die Konzentration der hierin beschriebenen Protease (aktives Enzym) in dem Mittel >0 bis 1 Gew.-%, vorzugsweise 0,001 bis 0,1 Gew.-% bezogen auf das Gesamtgewicht des Mittels oder der Zusammensetzung. Ferner kann die in dem Mittel enthaltene Protease, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Protease mit einer bei Raumtemperatur oder bei Abwesenheit von Wasser für die Protease undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

In weiteren Ausführungsformen der Erfindung ist das Mittel dadurch gekennzeichnet, dass es
(a) in fester Form vorliegt, insbesondere als rieselfähiges Pulver mit einem Schüttgewicht von 300 g/l bis 1200 g/l, insbesondere 500 g/l bis 900 g/l, oder
(b) in pastöser oder in flüssiger Form vorliegt, und/oder
(c) in gelförmiger oder dosierbeutelförmiger (Pouches) Form vorliegt, und/oder
(d) als Einkomponentensystem vorliegt, oder
(e) in mehrere Komponenten aufgeteilt ist.

Diese Ausführungsformen der vorliegenden Erfindung umfassen alle festen, pulverförmigen, flüssigen, gelförmigen oder pastösen Darreichungsformen erfindungsgemäßer Mittel, die gegebenenfalls auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwaschmittels oder einer nicht-wässrigen Paste oder in Form eines wässrigen Flüssigwaschmittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

Erfindungsgemäße Wasch- oder Reinigungsmittel können ausschließlich eine Protease enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Lipase, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder andere - von den erfindungsgemäßen Proteasen unterscheidbare - Protease, sowie deren Gemische. Weitere Enzyme sind in dem Mittel vorteilhafterweise jeweils in einer Menge von 1 × 10⁻⁸ bis 5 Gew.-% bezogen auf aktives Protein enthalten. Zunehmend bevorzugt ist jedes weitere Enzym in einer Menge von 1 × 10⁻⁷ bis 3 Gew.-%, von 0,00001 bis 1 Gew.-%, von 0,00005 bis 0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus vor zwischen der erfindungsgemäß enthaltenen Protease und einem weiteren Enzym eines erfindungsgemäßen Mittels, darunter insbesondere zwischen der genannten Protease und einer Amylase und/oder einer Lipase und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

In den hierin beschriebenen Reinigungsmitteln können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Die Enzyme können auch in wasserlösliche Filme, wie sie beispielsweise bei der Konfektionierung von Wasch- und Reinigungsmitteln in Einheitsdosisform verwendet werden, eingebracht werden. Ein derartiger Film ermöglicht die Freisetzung der Enzyme nach Kontakt mit Wasser. Wie hierin verwendet, bezieht sich "wasserlöslich" auf eine Filmstruktur, die vorzugsweise vollständig wasserlöslich ist. Bevorzugt besteht ein solcher Film aus (vollständig oder teilweise hydrolysiertem) Polyvinylalkohol (PVA).

Ein weiterer Erfindungsgegenstand ist ein Verfahren zur Reinigung von Textilien oder harten Oberflächen, das dadurch gekennzeichnet ist, dass in mindestens einem Verfahrensschritt ein erfindungsgemäßes Mittel angewendet wird, oder dass in mindestens einem Verfahrensschritt eine erfindungsgemäße Protease katalytisch aktiv wird, insbesondere derart, dass die Protease in einer Menge von 40µg bis 4g, vorzugsweise von 50µg bis 3g, besonders bevorzugt von 100µg bis 2g und ganz besonders bevorzugt von 200µg bis 1g eingesetzt wird.

In verschieden Ausführungsformen zeichnet sich das oben beschriebene Verfahren dadurch aus, dass die Protease bei einer Temperatur von 0 bis 100°C, bevorzugt 0 bis 60°C, weiter bevorzugt 20 bis 40°C und am meisten bevorzugt bei 20 oder 25°C eingesetzt wird.

Hierunter fallen sowohl manuelle als auch maschinelle Verfahren, wobei maschinelle Verfahren bevorzugt sind. Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Protease bereichert werden und stellen dann Ausführungsformen der vorliegenden Erfindung dar. Alle Sachverhalte, Gegenstände und Ausführungsformen, die für erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Da erfindungsgemäße Proteasen natürlicherweise bereits eine hydrolytische Aktivität besitzen und diese auch in Medien entfalten, die sonst keine Reinigungskraft besitzen wie beispielsweise in bloßem Puffer, kann ein einzelner und/oder der einzige Schritt eines solchen Verfahrens darin bestehen, dass als einzige reinigungsaktive Komponente eine erfindungsgemäße Protease mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Schließlich erfasst die Erfindung auch die Verwendung der hierin beschriebenen Proteasen in Wasch- oder Reinigungsmitteln, beispielsweise wie oben beschrieben, zur (verbesserten) Entfernung von proteinhaltigen Anschmutzungen, beispielsweise von Textilien oder harten Oberflächen. In bevorzugten Ausführungsformen dieser Verwendung wird die Protease im Wasch- oder Reinigungsmittel vor einem Wasch- oder Reinigungsvorgang 3 oder mehr Tage, 4 oder mehr Tage, 7 oder mehr Tage, 10 oder mehr Tage, 12 oder mehr Tage, 14 oder mehr Tage, 21 oder mehr Tage oder 28 oder mehr Tage gelagert.

Alle Sachverhalte, Gegenstände und Ausführungsformen, die für erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### Beispiele

### Übersicht über die Mutationen:

Diese Erfindung betrifft eine alkalische Protease vom Subtilisin Typ aus *Bacillus pumilus.* Von dieser Protease (SEQ ID NO:1), wurden durch Zufallsmutagenese Varianten hergestellt, welche dann u.a. auf verbesserte Waschleistung und/oder Enzymstabilität gescreent wurden. Auf diesem Weg wurden aus der oben genannten Wildtyp Protease in einer ersten Runde durch Error Prone Mutagenese zwei leistungsverbesserte Mutanten (Mutante 1 [SEQ ID NO:18] und Mutante 2 [SEQ ID NO:2]) generiert. Auf beide dieser Mutanten wurde eine unabhängige zweite Error Prone Runde aufgesetzt. In dieser zweiten Mutationsrunde wurden die Mutanten 3-17 gemäß SEQ ID Nos. 3-17 generiert. Deshalb tragen alle hier genannten Mutanten 3-17 auch mindestens einen Teil der Mutationen der Mutanten 1 oder 2. In einer dritten Runde wurde durch Error Prone Mutagenese eine weitere leistungsverbesserte Mutante (Mutante 18 [SEQ ID NO:19]) generiert. Auf dieser Mutante wurde eine vierte Error Prone Runde aufgesetzt. In dieser vierten Mutationsrunde wurden die Mutanten 19-23 gemäß SEQ ID Nos. 20-24 generiert. Deshalb tragen alle hier genannten Mutanten 19-23 auch die Mutationen der Mutante 18

| **Variante** | **Aminosäuresubstitutionen relativ zu SEQ ID NO:1** | | | | | | | | | **SEQ ID NO:** |
|---|---|---|---|---|---|---|---|---|---|---|
| Mutante 1 | P9T | Q271E | S216C | | | | | | | 18 |
| Mutante 2 | P9T | Q271E | S216C | T133R | S224A | | | | | 2 |
| Mutante 3 | P9T | Q271E | S216C | N130D | G131S | T133Y | S224A | | | 3 |
| Mutante 4 | P9T | Q271E | S216C | N130S | G131S | T133Y | N144L | S224A | | 4 |
| Mutante 5 | P9T | Q271E | S216C | N130D | G131N | T133K | N144k | | | 5 |
| Mutante 6 | P9T | Q271E | S216C | N130H | G131N | N144K | S224A | | | 6 |
| Mutante 7 | P9T | Q271E | S216C | A29G | N130D | G131N | T133R | | | 7 |
| Mutante 8 | P9T | Q271E | S216C | A29G | D101E | N130S | G131S | S224T | N252S | 8 |
| Mutante 9 | P9T | Q271E | S216C | N130H | G131S | S224A | | | | 9 |
| Mutante 10 | P9T | Q271E | S216C | N130D | G131S | T133K | S224A | | | 10 |
| Mutante 11 | P9T | Q271E | S216C | A29G | D101E | N130D | G131K | S224A | | 11 |
| Mutante 12 | P9T | Q271E | S216C | N130D | G131N | T133K | N144L | N252S | | 12 |
| Mutante 13 | P9T | Q271E | S216C | G131S | N144K | S224T | | | | 13 |
| Mutante 14 | P9T | Q271E | S216C | D101E | N130D | G131S | T133Y | N144A | S224A | 14 |
| Mutante 15 | P9T | Q271E | S216C | A29G | D101E | N130S | S224T | N252S | | 15 |
| Mutante 16 | P9T | Q271E | S216C | A48V | G131S | T133R | S224A | | | 16 |
| Mutante 17 | P9T | Q271E | S216C | G131D | T133R | S224A | | | | 17 |
| Mutante 18 | P9T | Q271E | | N130D | N144K | N252T | | | | 19 |
| Mutante 19 | P9T | Q271E | | N130D | N144K | N252T | G166M | | | 20 |
| Mutante 20 | P9T | Q271E | | N130D | N144K | N252T | G166Q | | | 21 |
| Mutante 21 | P9T | Q271E | | N130D | N144K | N252T | G166A | | | 22 |
| Mutante 22 | P9T | Q271E | | N130D | N144K | N252T | Y217M | | | 23 |
| Mutante 23 | P9T | Q271E | | N130D | N144K | N252T | S156G | | | 24 |
| Mutante 24 | P9T | Q271E | | N130D | N144K | N252T | D172P | | | |
| Mutante 25 | P9T | Q271E | | N130D | N144K | N252T | V149I | | | |
| Mutante 26 | P9T | Q271E | | N130D | N144K | N252T | Q62S | | | |
| Mutante 27 | P9T | Q271E | | N130D | N144K | N252T | Y217M | T133R | | |
| Mutante 28 | P9T | Q271E | | | N144K | N252T | Y217M | | | |
| Mutante 29 | P9T | Q271E | | N130D | N144K | N252T | S156Q | | | |

### Verwendete Waschmittelmatrix

Folgende Waschmittelmatrizen (handelsüblich, ohne Enzyme, opt. Aufheller, Parfüm und Farbstoffe) wurden für den Waschtest verwendet:

### Lagerstabilitätstest 1:

| **Chemischer Name** | Gew.-% Aktivsubstanz im Rohmaterial | Gew.-% Aktivsubstanz in der Formulierung |
|---|---|---|
| Wasser demin. | 100 | Rest |
| Alkylbenzolsulfonsäure | 96 | 4,4 |
| Anionische Tenside | 70 | 5,6 |
| C12-C18 Fettsäure Na-Salz | 30 | 2,4 |
| Nicht-ionische Tenside | 100 | 4,4 |
| Phosphonate | 40 | 0,2 |
| Zitronensäure | 100 | 1,4 |
| NaOH | 50 | 0,95 |
| Entschäumer | t.q. | 0,01 |
| Glycerin | 100 | 2 |
| Konservierungsmittel | 100 | 0,08 |
| Ethanol | 93 | 1 |
| Ohne opt. Aufheller, Parfüm, Farbstoff und Enzyme. | | |

| | | |
|---|---|---|
| Dosierung 4,7 g/L | | |

### Lagerstabilitätstest 2:

| **Chemischer Name** | Gew.-% Aktivsubstanz im Rohmaterial | Gew.-% Aktivsubstanz in der Formulierung |
|---|---|---|
| Wasser demin. | 100 | Rest |
| Alkylbenzolsulfonsäure | 96 | 12-18 |
| Anionische Tenside | 70 | 4-8 |
| C12-C18 Fettsäure Na-Salz | 30 | 2-4 |
| Nicht-ionische Tenside | 100 | 8-14 |
| Phosphonat | 60 | 0,5-2 |
| Zitronensäure | 100 | 3-5 |
| NaOH | 50 | 0,5-2 |
| Entschäumer | 100 | <1% |
| Glycerin | 99,5 | 1-3 |
| 1,2-Propandiol | 100 | 8-12 |
| Monoethanolamin | 100 | 4-8 |
| Soil repellent polymer | 30 | 0,5-1 |
| Ohne opt. Aufheller, Parfüm, Farbstoff und Enzyme. | | |

| | | |
|---|---|---|
| Dosierung 3,17 g/L | | |

### Protease-Aktivitätsassays

Die Aktivität der Protease wird durch die Freisetzung des Chromophors para-Nitroanilin aus dem Substrat Succinyl Alanin-Alanin-Prolin-Phenylalanin-para-Nitroanilid (AAPFpNA; Bachem L-1400) bestimmt. Die Freisetzung des pNA verursacht eine Zunahme der Extinktion bei 410 nm, deren zeitlicher Verlauf ein Maß für die enzymatische Aktivität ist.

Die Messung erfolgte bei einer Temperatur von 25°C, bei pH 8,6 und einer Wellenlänge von 410 nm. Die Messzeit betrug 5 min bei einem Messintervall von 20 bis 60 Sekunden.

### Messansatz:

10 µL AAPF-Lösung (70 mg/mL)
1000 µL Tris/HCl (0,1 M; pH 8,6 mit 0,1% Brij 35)
10 µL verdünnte Proteaselösung

Kinetik erstellt über 5 min bei 25°C (410 nm)

### Lagerstabilitätstest und Ergebnisse

Die Proteasen wurden auf gleichem Aktivitätslevel in eine Waschmittelmatrix eingerührt und bei 30°C gelagert. Mittels eines üblichen Aktivitätsassays für Proteasen (Hydrolyse von suc-AAPFpNA), wird die Startaktivität und die Restaktivität der Protease nach 4 Wochen Lagerung bei 30°C gemessen. Um harsche Bedingungen zu generieren wurden die Proteasen in eine Waschmittelmatrix ohne Stabilisator (Borsäure) gelagert.

Die Proteasen wurden in Schüttelkolben-Überständen aus *Bacillus subtilis* generiert. Sie wurden auf ein gleiches Aktivitätslevel verdünnt. Es wurde 50% Waschmittelmatrix ohne Borsäure mit 50% entsprechend verdünnten *Bacillus subtilis* Protease Überstand versetzt und gut durchmischt. Die verschlossenen Gläser wurden bei 30°C inkubiert. Zum Zeitpunkt der Probenahme wurde eine vorher bestimmte Menge Matrix/Proteasegemisch entnommen und für 20 min bei RT im Probenpuffer (0,1 M Tris/HCl, pH 8,6) durch Rühren gelöst. Dann wird der AAPF Assay wie oben beschrieben durchgeführt.

Im ersten Lagerstabilitätstest hatten sich 15 Mutanten als vorteilhaft herausgestellt. Die Aktivität ist in % der Restaktivität der Ausgangsvarianten (Mutante 1 gemäß SEQ ID NO:18 oder Mutante 2 gemäß SEQ ID NO:2) nach 4 Wochen Lagerung bei 30°C dargestellt. Die Mutanten 3-15 beziehen sich auf die Ausgangsmutante 1 (SEQ ID NO:18), die Mutanten 16 und 17 beziehen sich auf die Ausgangsmutante 2 (SEQ ID NO:2).

| **Variante** | **SEQ ID NO:** | **Restaktivität** |
|---|---|---|
| Mutante 1 | 18 | 100% |
| Mutante 3 | 3 | 153% |
| Mutante 4 | 4 | 148% |
| Mutante 5 | 5 | 150% |
| Mutante 6 | 6 | 133% |
| Mutante 7 | 7 | 154% |
| Mutante 8 | 8 | 132% |
| Mutante 9 | 9 | 147% |
| Mutante 10 | 10 | 146% |
| Mutante 11 | 11 | 132% |
| Mutante 12 | 12 | 144% |
| Mutante 13 | 13 | 136% |
| Mutante 14 | 14 | 138% |
| Mutante 15 | 15 | 132% |

| **Variante** | **SEQ ID NO:** | **Restaktivität** |
|---|---|---|
| Mutante 2 | 2 | 100% |
| Mutante 16 | 16 | 121% |
| Mutante 17 | 17 | 110% |

Es ist erkennbar, dass alle Mutanten eine stark verbesserte Stabilität ohne Zugabe von Borsäure im Vergleich zu den jeweiligen Ausgangsmutanten gemäß SEQ ID Nos. 18 und 2 zeigen. Alle Mutanten zeigen eine vergleichbare Waschleistung wie der Wildtyp gemäß SEQ ID NO:1, d.h. sie sind maximal 10% schlechter in der Waschleistung, was im Rahmen der Messschwankung liegt (Ergebnisse nicht gezeigt).

Im zweiten Lagerstabilitätstest hatten sich 6 Mutanten als vorteilhaft herausgestellt. Die Aktivität ist in % der Restaktivität der Ausgangsvariante (Mutante 18 gemäß SEQ ID NO:19) - die bereits eine deutlich verbesserte Stabilität gegenüber dem Wildtypenzym aufweist - nach 4 Wochen Lagerung bei 30°C dargestellt.

| **Variante** | **SEQ ID NO:** | **Restaktivität** |
|---|---|---|
| Mutante 18 | 19 | 100% |
| Mutante 19 | 20 | 117% |
| Mutante 20 | 21 | 118% |
| Mutante 21 | 22 | 107% |
| Mutante 22 | 23 | 109% |
| Mutante 23 | 24 | 109% |
| Mutante 29 | | 117% |

Es ist erkennbar, dass alle Mutanten ohne Zugabe von Borsäure im Vergleich zu der Ausgangsmutante gemäß SEQ ID NO:19 eine verbesserte Stabilität zeigen. Alle Mutanten zeigen eine vergleichbare Waschleistung wie der Wildtyp gemäß SEQ ID NO:1, d.h. sie sind maximal 10% schlechter in der Waschleistung, was im Rahmen der Messschwankung liegt (Ergebnisse nicht gezeigt).

In einer weiteren Testrunde wurden weitere Mutanten auf ihre Stabilität getestet, wobei die Inkubation und Lagerung wie oben beschrieben, allerdings bei 40°C, erfolgte.

Folgende Mutanten haben sich als vorteilhaft herausgestellt. Die Aktivität ist in % der Restaktivität zum Anfangswert dargestellt. Die Ausgangsvariante (Mutante 18 gemäß SEQ ID NO:19) weist bereits eine deutlich verbesserte Stabilität gegenüber dem Wildtypenzym aufweist. Gelagert wurde für 4 Wochen bei 40°C.

| **Variante** | **SEQ ID NO:** | **Restaktivität** |
|---|---|---|
| Mutante 18 | 19 | 10% |
| Mutante 24 | | 19% |
| Mutante 25 | | 22% |
| Mutante 26 | | 39% |
| Mutante 27 | | 28% |
| Mutante 28 | | 16% |

Alle Mutanten zeigten eine weitere Verbesserung der Stabilität.

## Patentansprüche

1. Protease umfassend eine Aminosäuresequenz, die mindestens 80% Sequenzidentität mit der in SEQ ID NO:1 angegebenen Aminosäuresequenz über deren Gesamtlänge aufweist und jeweils bezogen auf die Nummerierung gemäß SEQ ID NO:1 Aminosäuresubstitutionen
(a) an den Positionen, die den Positionen 9 und 271, entsprechen, und
(b) an mindestens drei der Positionen, die den Positionen 29, 48, 101, 130, 131, 133, 144, 224 oder 252, jeweils bezogen auf die Nummerierung gemäß SEQ ID NO:1, entsprechen, aufweist,
wobei
(1) die Aminosäuresubstitutionen an den Positionen, die den Positionen 9 und 271 entsprechen, die Aminosäuresubstitutionen P9T, P9H, P9S oder P9A und Q271E sind; und
(2) die mindestens drei Aminosäuresubstitutionen gemäß (b) aus der Gruppe bestehend aus A29G, A48V, D101E, N130D, N130S, N130H, G131S, G131N, G131D, G131K, T133K, T133R, T133Y, N144K, N144L, N144A, S224A, S224T oder N252S, jeweils bezogen auf die Nummerierung gemäß SEQ ID NO:1, ausgewählt sind.

2. Protease nach Anspruch 1, wobei die Protease Aminosäuresubstitutionen
(1) an den Positionen, die den Positionen 9 und 271 sowie mindestens drei von 130, 131, 133, 144 und 224, jeweils bezogen auf die Nummerierung gemäß SEQ ID NO:1, entsprechen, und
(2) optional an mindestens einer der Positionen, die den Positionen 29, 48, 101 und 252, jeweils bezogen auf die Nummerierung gemäß SEQ ID NO:1, entsprechen, aufweist.

3. Protease nach einem der Ansprüche 1-2, wobei die Protease eine der folgenden Aminosäuresubstitutionsvarianten, jeweils bezogen auf die Nummerierung gemäß SEQ ID NO:1, aufweist:
P9T, P9H, P9S oder P9A, insbesondere P9T, und Q271E und optional S216C sowie zusätzliche eine von
(i) N130D, G131S, T133Y und S224A;
(ii) N130S, G131S, T133Y, N144L und S224A;
(iii) N130D, G131N, T133K und N144K;
(iv) N130H, G131N, N144K und S224A;
(v) A29G, N130D, G131N und T133R;
(vi) A29G, D101E, N130S, G131S, S224T und N252S;
(vii) N130H, G131S und S224A;
(viii) N130D, G131S, T133K und S224A;
(ix) A29G, D101E, N130D, G131K und S224A;
(x) N130D, G131N T133K, N144L und N252S;
(xi) G131S, N144K und S224T;
(xii) D101E, N130D, G131S, T133Y, N144A und S224A;
(xiii) A29G, D101E, N130S, S224T und N252S;
(xiv) A48V, G131S, T133R und S224A; oder
(xv) G131D, T133R und S224A.

4. Protease nach einem der Ansprüche 1 bis 2, wobei die Protease bezogen auf die Nummerierung gemäß SEQ ID NO:1
(a) an den Positionen, die den Positionen 9, 130, 144, 252 und 271 entsprechen, die Aminosäuresubstitutionen 9T, 130D, 144K, 252T und 271E aufweist; sowie optional
(b1) an der Position, die der Position 166 entspricht, die Aminosäuresubstitution 166M, 166Q oder 166A; und/oder
(b2) an mindestens einer der Positionen, die den Positionen 62, 133, 149, 156, 172 oder 217 entsprechen, mindestens eine weitere Aminosäuresubstitution;
aufweist.

5. Protease nach Anspruch 4, wobei die Aminosäuresubstitutionen an den Positionen, die den Positionen 62, 133, 149, 156, 172 oder 217 entsprechen, ausgewählt sind aus den Aminosäuresubstitutionen 62S, 133R, 133A, 133K, 149I, 156G, 156Q, 172P, 172G und 217M.

6. Protease nach einem der Ansprüche 4-5, wobei die Protease
(1) an den Positionen, die den Positionen 9, 130, 144, 252 und 271 entsprechen, die Aminosäuresubstitutionen 9T, 130D, 144K, 252T und 271E; und
(2) an einer der Positionen, die den Positionen 62, 133, 149, 156, 166, 172 oder 217, entsprechen, eine der Aminosäuresubstitutionen 62S, 133R, 133A, 133K, 149I, 156G, 156Q, 166M, 166Q, 166A, 172P, 172G oder 217M;
aufweist.

7. Protease nach einem der Ansprüche 1-6, wobei die Protease eine Aminosäuresequenz gemäß SEQ ID Nos. 3-17 oder 19-23 aufweist.

8. Nukleinsäure kodierend für eine Protease nach einem der Ansprüche 1-7.

9. Vektor enthaltend eine Nukleinsäure nach Anspruch 8, insbesondere ein Klonierungsvektor oder ein Expressionsvektor.

10. Nicht-menschliche Wirtszelle, die eine Nukleinsäure nach Anspruch 8 oder einen Vektor nach Anspruch 9 beinhaltet, oder die eine Protease nach einem der Ansprüche 1-7 beinhaltet.

11. Verfahren zur Herstellung einer Protease umfassend
a) Kultivieren einer Wirtszelle gemäß Anspruch 10; und
b) Isolieren der Protease aus dem Kulturmedium oder aus der Wirtszelle.

12. Mittel, insbesondere ein Wasch- oder Reinigungsmittel, **dadurch gekennzeichnet, dass** es mindestens eine Protease nach einem der Ansprüche 1-7 enthält.

13. Verfahren zur Reinigung von Textilien oder harten Oberflächen, **dadurch gekennzeichnet, dass** in mindestens einem Verfahrensschritt ein Mittel nach Anspruch 12 angewendet wird, oder dass in mindestens einem Verfahrensschritt eine Protease nach einem der Ansprüche 1-7 angewendet wird.

14. Verwendung einer Protease nach einem der Ansprüche 1-7 in einem Wasch- oder Reinigungsmittel zur Entfernung von peptid- oder proteinhaltigen Anschmutzungen.

## Claims

1. A protease comprising an amino acid sequence which has at least 80% sequence identity with the amino acid sequence specified in SEQ ID NO:1 over the entire length thereof and amino acid substitutions, in each case based on the numbering according to SEQ ID NO:1,
(a) at the positions corresponding to positions 9 and 271, and
(b) at least three of the positions corresponding to positions 29, 48, 101, 130, 131, 133, 144, 224 or 252, in each case based on the numbering according to SEQ ID NO:1,
wherein
(1) the amino acid substitutions at the positions corresponding to positions 9 and 271 are the amino acid substitutions P9T, P9H, P9S or P9A and Q271E; and
(2) the at least three amino acid substitutions according to (b) are selected from the group consisting of A29G, A48V, D101E, N130D, N130S, N130H, G131S, G131N, G131D, G131K, T133K, T133R, T133Y, N144K, N144L, N144A, S224A, S224T or N252S, in each case based on the numbering according to SEQ ID NO:1.

2. The protease of claim 1, wherein the protease comprises amino acid substitutions
(1) at the positions corresponding to positions 9 and 271 as well as at least three of 130, 131, 133, 144 and 224, in each case based on the numbering according to SEQ ID NO:1, and
(2) optionally at at least one of the positions corresponding to positions 29, 48, 101 and 252, in each case based on the numbering according to SEQ ID NO:1.

3. The protease according to any one of claims 1-2, wherein the protease comprises one of the following amino acid substitution variants, in each case based on the numbering according to SEQ ID NO:1:
P9T, P9H, P9S or P9A, in particular P9T, and Q271E and optionally S216C as well as an additional one of
(i) N130D, G131S, T133Y and S224A;
(ii) N130S, G131S, T133Y, N144L and S224A;
(iii) N130D, G131N, T133K and N144K;
(iv) N130H, G131N, N144K and S224A;
(v) A29G, N130D, G131N and T133R;
(vi) A29G, D101E, N130S, G131S, S224T and N252S;
(vii) N130H, G131S and S224A;
(viii) N130D, G131S, T133K and S224A;
(ix) A29G, D101E, N130D, G131K and S224A;
(x) N130D, G131N T133K, N144L and N252S;
(xi) G131S, N144K and S224T;
(xii) D101E, N130D, G131S, T133Y, N144A and S224A;
(xiii) A29G, D101E, N130S, S224T and N252S;
(xiv) A48V, G131S, T133R and S224A; or
(xv) G131D, T133R and S224A.

4. The protease according to any one of claims 1 to 2, wherein the protease, based on the numbering according to SEQ ID NO:1, has
(a) at the positions corresponding to positions 9, 130, 144, 252 and 271, the amino acid substitutions 9T, 130D, 144K, 252T and 271E; as well as optionally
(b1) at the position corresponding to position 166, the amino acid substitution 166M, 166Q or 166A; and/or
(b2) at at least one of the positions corresponding to positions 62, 133, 149, 156, 172 or 217, at least one further amino acid substitution.

5. The protease of claim 4, wherein the amino acid substitutions at the positions corresponding to positions 62, 133, 149, 156, 172 or 217 are selected from the amino acid substitutions 62S, 133R, 133A, 133K, 149I, 156G, 156Q, 172P, 172G and 217M.

6. The protease according to any one of claims 4-5, wherein the protease has
(1) at the positions corresponding to positions 9, 130, 144, 252 and 271, the amino acid substitutions 9T, 130D, 144K, 252T and 271E; and
(2) at one of the positions corresponding to positions 62, 133, 149, 156, 166, 172 or 217, one of the amino acid substitutions 62S, 133R, 133A, 133K, 149I, 156G, 156Q, 166M, 166Q, 166A, 172P, 172G or 217M.

7. The protease according to any one of claims 1-6, wherein the protease has an amino acid sequence according to SEQ ID Nos. 3-17 or 19-23.

8. A nucleic acid encoding a protease according to any one of claims 1-7.

9. A vector comprising a nucleic acid according to claim 8, in particular a cloning vector or an expression vector.

10. A non-human host cell comprising a nucleic acid according to claim 8 or a vector according to claim 9, or comprising a protease according to any one of claims 1-7.

11. Method for the preparation of a protease comprising
a) culturing a host cell according to claim 10; and
b) Isolation of the protease from the culture medium or from the host cell.

12. Agent, in particular a washing or cleaning agent, **characterized in that** it contains at least one protease according to one of claims 1-7.

13. Method for cleaning textiles or hard surfaces, **characterized in that** an agent according to claim 12 is used in at least one method step, or **in that** a protease according to one of claims 1-7 is used in at least one method step.

14. Use of a protease according to any one of claims 1-7 in a washing or cleaning agent for removing peptide- or protein-containing soiling.

## Revendications

1. Protéase comprenant une séquence d'acides aminés ayant une identité de séquence d'au moins 80 % avec la séquence d'acides aminés spécifiée dans SEQ ID NO:1 sur toute sa longueur et des substitutions d'acides aminés, dans chaque cas sur la base de la numérotation selon SEQ ID NO:1,
(a) aux positions correspondant aux positions 9 et 271, et
(b) à au moins trois des positions correspondant aux positions 29, 48, 101, 130, 131, 133, 144, 224 ou 252, dans chaque cas sur la base de la numérotation selon SEQ ID NO:1,
dans lequel
(1) les substitutions d'acides aminés aux positions correspondant aux positions 9 et 271 sont les substitutions d'acides aminés P9T, P9H, P9S ou P9A et Q271E ; et
(2) les trois substitutions d'acide aminé au moins visées au point b) sont choisies dans le groupe constitué par A29G, A48V, D101E, N130D, N130S, N130H, G131S, G131N, G131D, G131K, T133K, T133R, T133Y, N144K, N144L, N144A, S224A, S224T ou N252S, dans chaque cas sur la base de la numérotation selon SEQ ID NO:1.

2. La protéase de la revendication 1, dans laquelle la protéase comprend des substitutions d'acides aminés
(1) aux positions correspondant aux positions 9 et 271 ainsi qu'à au moins trois des positions 130, 131, 133, 144 et 224, dans chaque cas sur la base de la numérotation selon SEQ ID NO:1, et
(2) éventuellement à au moins une des positions correspondant aux positions 29, 48, 101 et 252, dans chaque cas sur la base de la numérotation selon SEQ ID NO: 1.

3. La protéase selon l'une des revendications 1-2, dans laquelle la protéase comprend l'un des variants de substitution d'acide aminé suivants, dans chaque cas basé sur la numérotation selon SEQ ID NO:1 :
P9T, P9H, P9S ou P9A, en particulier P9T, et Q271E et éventuellement S216C, ainsi qu'un autre des éléments suivants
(i) N130D, G131S, T133Y et S224A ;
(ii) N130S, G131S, T133Y, N144L et S224A ;
(iii) N130D, G131N, T133K et N144K ;
(iv) N130H, G131N, N144K et S224A ;
(v) A29G, N130D, G131N et T133R ;
(vi) A29G, D101E, N130S, G131S, S224T et N252S ;
(vii) N130H, G131S et S224A ;
(viii) N130D, G131S, T133K et S224A ;
(ix) A29G, D101E, N130D, G131K et S224A ;
(x) N130D, G131N T133K, N144L et N252S ;
(xi) G131S, N144K et S224T ;
(xii) D101E, N130D, G131S, T133Y, N144A et S224A ;
(xiii) A29G, D101E, N130S, S224T et N252S ;
(xiv) A48V, G131S, T133R et S224A ; ou
(xv) G131D, T133R et S224A.

4. La protéase selon l'une des revendications 1 à 2, dans laquelle la protéase, sur la base de la numérotation selon SEQ ID NO:1, a
(a) aux positions correspondant aux positions 9, 130, 144, 252 et 271, les substitutions d'acides aminés 9T, 130D, 144K, 252T et 271E ; ainsi que, éventuellement, (b1) à la position correspondant aux positions 9, 130, 144K, 252T et 271E, la substitution de l'acide aminé 9T.
(b1) à la position correspondant à la position 166, la substitution d'acide aminé 166M, 166Q ou 166A ; et/ou
(b2) à au moins une des positions correspondant aux positions 62, 133, 149, 156, 172 ou 217, au moins une autre substitution d'acide aminé.

5. La protéase de la revendication 4, dans laquelle les substitutions d'acides aminés aux positions correspondant aux positions 62, 133, 149, 156, 172 ou 217 sont choisies parmi les substitutions d'acides aminés 62S, 133R, 133A, 133K, 149I, 156G, 156Q, 172P, 172G et 217M.

6. Protéase selon l'une des revendications 4 à 5, dans laquelle la protéase possède
(1) aux positions correspondant aux positions 9, 130, 144, 252 et 271, les substitutions d'acides aminés 9T, 130D, 144K, 252T et 271E ; et
(2) à l'une des positions correspondant aux positions 62, 133, 149, 156, 166, 172 ou 217, l'une des substitutions d'acide aminé 62S, 133R, 133A, 133K, 149I, 156G, 156Q, 166M, 166Q, 166A, 172P, 172G ou 217M.

7. La protéase selon l'une des revendications 1 à 6, dans laquelle la protéase a une séquence d'acides aminés selon les numéros SEQ ID 3 à 17 ou 19 à 23.

8. Acide nucléique codant pour une protéase selon l'une des revendications 1 à 7.

9. Vecteur comprenant un acide nucléique selon la revendication 8, en particulier un vecteur de clonage ou un vecteur d'expression.

10. Cellule hôte non humaine comprenant un acide nucléique selon la revendication 8 ou un vecteur selon la revendication 9, ou comprenant une protéase selon l'une des revendications 1 à 7.

11. Méthode de préparation d'une protéase comprenant
a) la culture d'une cellule hôte selon la revendication 10 ; et
b) l'isolement de la protéase à partir du milieu de culture ou de la cellule hôte.

12. Agent, en particulier agent de lavage ou de nettoyage, **caractérisé en ce qu'**il contient au moins une protéase selon l'une des revendications 1 à 7.

13. Méthode de nettoyage de textiles ou de surfaces dures, **caractérisée en ce qu'**un agent selon la revendication 12 est utilisé dans au moins une étape de la méthode, ou **en ce qu'**une protéase selon l'une des revendications 1-7 est utilisée dans au moins une étape de la méthode.

14. Utilisation d'une protéase selon l'une des revendications 1 à 7 dans un agent de lavage ou de nettoyage pour éliminer les salissures contenant des peptides ou des protéines.
